(19) Europäisches Patentamt
European Patent Office
Office européen des brevets

(11) EP 4 554 928 B1

(12) EUROPEAN PATENT SPECIFICATION

(45) Date of publication and mention of the grant of the patent:
**17.06.2026 Bulletin 2026/25**

(21) Application number: **23741054.3**

(22) Date of filing: **12.07.2023**

(51) International Patent Classification (IPC):
***C07D 207/30*** (2006.01) ***C08K 3/04*** (2006.01)
***C08K 5/3415*** (2006.01) ***C08L 7/00*** (2006.01)
***C09C 1/44*** (2006.01) ***B60C 1/00*** (2006.01)
***C07D 207/333*** (2006.01) ***C08L 9/00*** (2006.01)
***C08K 3/36*** (2006.01)

(52) Cooperative Patent Classification (CPC):
(C-Sets available)
**C08L 7/00; B60C 1/00; C07D 207/34; C08K 3/04; C08K 5/3415; C09C 1/56;** C01P 2002/88 (Cont.)

(86) International application number:
**PCT/EP2023/069355**

(87) International publication number:
**WO 2024/013246 (18.01.2024 Gazette 2024/03)**

(54) **ELASTOMER COMPOSITIONS COMPRISING AT LEAST ONE SP2 HYBRIDIZED CARBON ALLOTROPE AND A 2,5-DISUBSTITUTED PYRROLE DERIVATIVE**

ELASTOMERZUSAMMENSETZUNGEN MIT MINDESTENS EINEM SP2-HYBRIDISIERTEN KOHLENSTOFFALLOTROP UND EINEM 2,5-DISUBSTITUIERTEN PYRROLDERIVAT

COMPOSITIONS ÉLASTOMÈRES COMPRENANT AU MOINS UN ALLOTROPE DE CARBONE HYBRIDÉ SP2 ET UN DÉRIVÉ DE PYRROLE 2,5-DISUBSTITUÉ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **13.07.2022 IT 202200014752**

(43) Date of publication of application:
**21.05.2025 Bulletin 2025/21**

(73) Proprietor: **Pirelli Tyre S.p.A.**
**20126 Milano (IT)**

(72) Inventors:
- **GALIMBERTI, Maurizio Stefano**
  **20133 MILANO (IT)**
- **BARBERA, Vincenzina**
  **20133 MILANO (IT)**
- **MARGANI, Fatima**
  **20133 MILANO (IT)**
- **GIANNINI, Luca**
  **20126 MILANO (IT)**

(74) Representative: **Merli, Silvia et al**
**Marchi & Partners S.r.l.**
**Via Vittor Pisani, 13**
**20124 Milano (IT)**

(56) References cited:
**WO-A1-2016/050887** **WO-A1-2020/225595**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C08L 7/00, C08L 9/00, C08K 3/04, C08K 5/548, C08K 3/36, C08K 3/22, C08K 5/09, C08K 5/18, C08K 3/06, C08K 5/47, C08K 5/44, C08K 5/3415**

## Description

The present invention generally relates to elastomer compositions comprising at least one $sp^2$ hybridized carbon allotrope and a 2,5-disubstituted pyrrole derivative, preferably provided as a pre-formed adduct, the process for their preparation and tyres comprising such elastomer compositions. The present invention further relates to adducts between said pyrrole derivatives and $sp^2$ hybridized carbon allotropes as well as to the process for their preparation.

## PRIOR ART

The peculiar feature of elastomeric composites is the entropic elasticity, which occurs above the glass transition temperature of the elastomers and is at the basis of their performances. Diene elastomers, i.e. elastomers with unsaturation in the backbone chain such as poly(1,4-cis-butadiene) and poly(1,4-cis-isoprene), are characterized by a great mobility of the backbone chain, due to the easy rotation of the polymer chain around the single bonds close to the unsaturations. Upon crosslinking the polymer chains of these unsaturated elastomers, the composite acquires the property of entropic elasticity. However, despite the outstanding elasticity, elastomeric composites do not have the mechanical properties required for demanding applications, such as the one in tyre compounds. To achieve such properties, the composites have to be loaded with reinforcing fillers.

Since the beginning of the twentieth century, carbon black has been used as reinforcing filler for elastomeric composites. Thanks to the addition of carbon black, both the static and dynamic-mechanical properties of elastomeric composites increase. However, carbon black brings also about a remarkable increase of the hysteresis, hence of the dissipation of energy, of the elastomeric composites. Indeed, it is known that the elastic modulus of a composite material filled with carbon black, to which sinusoidal stresses have been applied, decreases, passing from minimum strain up to around 25% of strain (limit estimated for linear behaviour). This phenomenon is known as the "Payne Effect" and is an indicator of the energy dissipation of the material.

To have mechanical reinforcement and low dissipation of energy, silica is used as reinforcing filler, in place of or together with carbon black. Thanks to the use of a coupling agent, typically a silane containing sulphur atoms, a chemical bond is established between silica and the elastomers' chains, and this leads to the reduction of hysteresis and of the dissipation of energy. Hence, the use of silica has been dramatically increasing over the last decades, despite the drawbacks connected to its use.

Indeed, silica leads to the increase of the compound viscosity, to the worsening of processability and to the shortening of the storage time of the composites. Such drawbacks arise from the surface activity of silica: the polar groups promote extensive supramolecular interactions. Of note, a shorter storage time results in the need for a specific planning for the compounds' production and the procedures for storing and moving the compounds, with a clear impact on the logistics.

Moreover, to achieve efficient mixing of silica-based composites, specific expensive mixing equipment are required. Due to the use of a silane, silica-based compounds have increased adhesiveness to the metal parts of the mixing machines, and this requires special treatments of the metal surfaces. Finally, silica is corrosive and abrasive and this as well requires special treatments of the metal surfaces and the revision of the maintenance procedures.

These drawbacks are all relevant at the industrial scale, for example when silica based elastomeric composites are used for tyre compounds. However, tyre compounds, particularly those used for tyre treads, are typically almost exclusively based on silica as the reinforcing filler and increasing research efforts are made to use silica in partial replacement of carbon black, also in tyre compounds other than the tyre tread. Research efforts are also made to use silica as the only filler in tyre compounds other than the tyre tread. This is because the prevailing objective is the reduction of energy dissipation of a rolling tyre and this objective has never been achieved, at least in the existing prior art, with carbon black as the sole reinforcing filler.

Indeed, several attempts at reducing the Payne effect due to carbon black have been made, for example by optimizing its dispersion in the elastomer matrix, separating the aggregates and/or the elemental particles from it, covering them with a layer of elastomer.

The Applicants have already reported different approaches to achieve this (see e.g. WO 2016/050887, WO 2020/225595A1, WO 2020/222103). However no attempt at partially, or completely, replace silica has been reported so far.

## SUMMARY OF THE INVENTION

The Applicants addressed the need to obtain elastomer compositions overcoming, at least in part, the drawbacks described above.

In particular, the Applicants have tackled the problem of providing elastomer compositions endowed with the dynamic-mechanical properties usually obtained by using silica as reinforcing filler and at the same time the processability

and the abovementioned advantages linked to the use of carbon black.

The Applicants have also addressed the problem to obtain such elastomer compositions by means of simple techniques, with reduced environmental impact, and using the standard solid state mixing techniques used in the tyres industry.

Surprisingly, the Applicants have found that the use of at least one $sp^2$ hybridized carbon allotrope and a 2,5-disubstituted pyrrole derivative, preferably provided as a pre-formed adduct between each other, as reinforcing fillers in elastomeric compositions is able to achieve a balance between the advantages brought about by both silica and carbon black, while at the same time overcoming the relating drawbacks.

The elastomer compositions thus obtained exhibit low hysteresis and a reduced Payne effect, with consequent reduced dissipation of energy, and are thus particularly suitable as compounds for tyres.

Thus, according to a first aspect, the invention relates to an elastomer composition comprising at least one $sp^2$ hybridized carbon allotrope and a compound of formula (I)

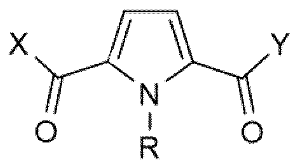

(I)

wherein X and Y are independently selected from the group consisting of:
-NHR' and OR"; and

wherein R" is selected from the group consisting of hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, and heteroaryl; and
wherein R and R' are independently selected from the group consisting of: hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, and carboxyl;
or R and/or R' is

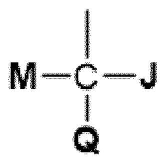

wherein M, Q and J are independently selected in a group consisting of amine, hydroxyl, hydrogen, linear or branched C1-C10 alkyl, and linear or branched C2-C10 alkenyl or alkynyl, or in a group consisting of:

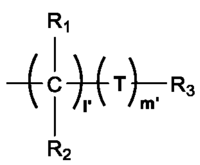 , 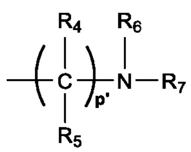 , 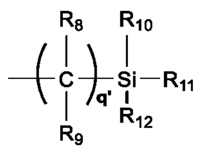 , 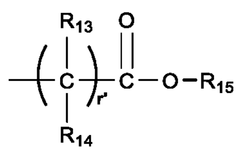 ,

wherein $R_1$-$R_{15}$ are independently selected from the group consisting of: hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, and carboxyl;
and wherein $R_{10}$-$R_{12}$ can be $-OCH_2-CH_3$, or $-OCH_3$;
and wherein $R_{14}$ can be $-CH_2-SH$, or $-CH_2-CH_2-S-CH_3$;
and wherein T is -O- or -S-, and when T is -S- then p' is an integer from 1 to 4, when T is -O- then p' is an integer from 1 to 2;
and wherein l', p', q', r' are independently integers from 0 to 12;
or R and/or R' is

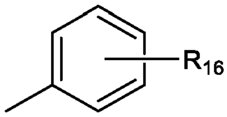 or 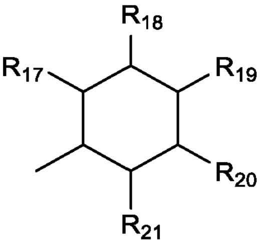 ,

wherein $R_{16}$ is selected from the group consisting of hydrogen, alkyl, aryl, benzyl, amine, alkylamine, arylamine, benzylamine, and aminoaryl; and
$R_{17}$-$R_{21}$ are independently selected from the group consisting of: hydrogen, linear or branched C1-C10 alkyl, linear or branched C2-C10 alkenyl or alkynyl, and 1- (4-aminocyclohexyl) methylene.

In an embodiment of the invention, the elastomer composition comprises a pre-formed adduct between said compound of formula (I) and said at least one $sp^2$ hybridized carbon allotrope, that is to mean that said compound of formula (I) and said at least one $sp^2$ hybridized carbon allotrope are provided as a pre-formed adduct.
In a second aspect, the present invention relates to a process for the preparation of an elastomer composition as defined above, comprising:

a) providing one or more elastomers, at least one $sp^2$ hybridized carbon allotrope, a compound of formula (I) as defined above, optionally one or more additional reinforcing fillers, optionally one or more additives, and optionally one or more vulcanizing agents; and
b) performing at least one mixing step.

In a third aspect, the invention relates to a tyre for vehicle wheels comprising at least one structural element comprising a crosslinked elastomer material obtained by crosslinking of an elastomer composition as defined above.
Finally, in a fourth and fifth aspects, the present invention further relates to an adduct between a $sp^2$ hybridized carbon allotrope and a compound of formula (I) as defined above, and, respectively, to a process for the preparation of said adduct, the process comprising the steps of:

i) providing a mixture of the compound of formula (I) and the $sp^2$ hybridized carbon allotrope; and
ii) supplying energy to the mixture obtained in step i) to obtain an adduct; and
iii) optionally separating the adduct obtained.

## BRIEF DESCRIPTION OF THE FIGURES

The description is illustrated here with reference to the attached drawings, provided solely by way of example and not limiting the invention.

Figure 1: outline of the transverse half-section of a tyre comprising in one or more components the elastomer composition according to the present invention.
Figure 2a-2b: exemplary TGA spectrum, respectively before and after the purification procedure, of an adduct according to the invention.
Figure 3: graph showing the variation of G' (y axis) versus the deformation amplitude (x axis) for the elastomer compositions of examples 6-10.
Figure 4: graph showing the variation of tan delta (y axis) versus the deformation (x axis) for the elastomer compositions of examples 6-10.

## DETAILED DESCRIPTION OF THE INVENTION

*Definitions*

According to the present invention, the term "adduct" is understood to mean a compound obtained by combining two or more components; more specifically, the term adduct indicates compounds whose components, bound more or less loosely, i.e. via covalent bonds or by means of more labile intermolecular interactions, retain their individuality in some ways. In particular according to the present invention, the term "adduct(s)" refer to adducts obtained from the interaction, via covalent or noncovalent bonds, between a pyrrole derivative, i.e. the compound of formula (I) as defined above, and a

$sp^2$ hybridized carbon allotrope.

According to the present description, the terms "carbon allotrope" and "carbon-based filler" are used interchangeably, and/or are both indicated with the abbreviation CA.

In the following, the term "elastomer composition" is used to include the totality of all the components which are added in the preparation of the elastomer mixture, independently of the fact that these may all actually be present simultaneously, that they may be introduced sequentially or that they may then be traceable in the elastomer mixture or in the final tyre.

For the purposes of the present description and the following claims, the term "phr" (parts per hundreds of rubber) indicates the parts by weight of a defined component of the elastomer composition per 100 parts by weight of the elastomer polymer.

For the purposes of the present description and the following claims, the expression "elastomer composition comprising at least one $sp^2$ hybridized carbon allotrope and a compound of formula (I)" is intended to mean that the elastomer composition according to the present invention can contain said at least one $sp^2$ hybridized carbon allotrope and said compound of formula (I) either as distinct ingredients or as a pre-formed adduct between the two.

For the purposes of the present description and the following claims, the expression "providing [...] at least one $sp^2$ hybridized carbon allotrope, a compound of formula (I) [...]" is intended to mean that, in the process for the preparation of an elastomer composition according to the present invention, said at least one $sp^2$ hybridized carbon allotrope and said compound of formula (I) can be provided either as distinct ingredients or as a pre-formed adduct between the two.

For the purposes of the present description and the following claims, the compounds of formula (I) and (II) herein disclosed also include derivatives, such as esters, salts, enantiomers, diastereomers, preferably esters and salts.

*Detailed description*

In a first aspect, the present invention relates to an elastomer composition comprising at least one $sp^2$ hybridized carbon allotrope and a compound of formula (I)

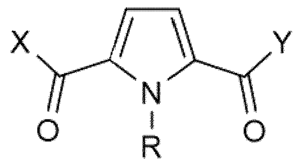

(I)

wherein X and Y are independently selected from the group consisting of:
-NHR' and OR"; and
wherein R" is selected from the group consisting of hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, and heteroaryl; and
wherein R and R' are independently selected from the group consisting of: hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, and carboxyl;
or R and/or R' is

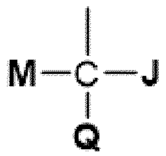

wherein M, Q and J are independently selected in a group consisting of amine, hydroxyl, hydrogen, linear or branched C1-C10 alkyl, and linear or branched C2-C10 alkenyl or alkynyl, or in a group consisting of:

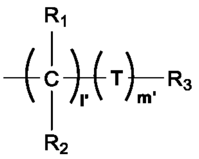 , 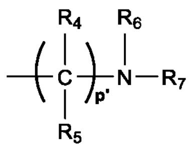 , 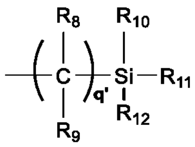 , 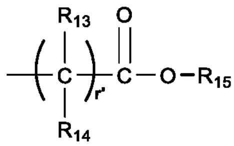 ,

wherein $R_1$-$R_{15}$ are independently selected from the group consisting of: hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, and carboxyl;
and wherein $R_{10}$-$R_{12}$ can be $-OCH_2-CH_3$, or $-OCH_3$;
and wherein $R_{14}$ can be $-CH_2-SH$, or $-CH_2-CH_2-S-CH_3$;
and wherein T is -O- or -S-, and when T is -S- then p' is an integer from 1 to 4, when T is -O- then p' is an integer from 1 to 2;
and wherein l', p', q', r' are independently integers from 0 to 12;
or R and/or R' is

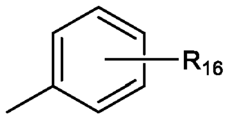 or 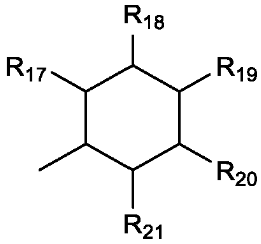 ,

wherein $R_{16}$ is selected from the group consisting of hydrogen, alkyl, aryl, benzyl, amine, alkylamine, arylamine, benzylamine, and aminoaryl; and
$R_{17}$-$R_{21}$ are independently selected from the group consisting of: hydrogen, linear or branched C1-C10 alkyl, linear or branched C2-C10 alkenyl or alkynyl, and 1- (4-aminocyclohexyl) methylene.

In a preferred embodiment, said R and R' are the same.

Preferably, said R and R' are independently selected from the group consisting of hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, carboxyl, linear or branched C1-C10 alkyl-$NH_2$, linear or branched C1-C10 alkyl-SH, linear or branched C1-C10 alkyl-COOH, and linear or branched C1-C10 alkyl-$Si(OCH_2CH_3)_3$, wherein said alkyl groups are optionally substituted with one or more - OH groups. In a preferred embodiment, said R and R' are the same.

In a preferred embodiment, said compound of formula (I) is represented by formula (II) below

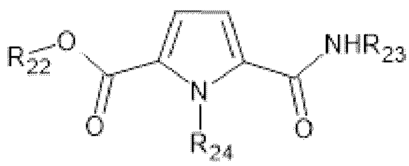
(II)

wherein said $R_{22}$ is selected from the group consisting of hydrogen and C1-C10 linear or branched alkyl group optionally substituted with one or more -OH groups; and said $R_{23}$-$R_{24}$ are the same and are selected from the group consisting of hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, linear or branched C1-C10 alkyl-$NH_2$, linear or branched C1-C10 alkyl-SH, linear or branched C1-C10 alkyl-COOH, and linear or branched C1-C10 alkyl-$Si(OCH_2CH_3)_3$, wherein said alkyl groups are optionally substituted with one or more -OH groups.

In particularly preferred embodiments of the present invention, said compound of formula (I) is selected from the group consisting of:

- 1-(1,3-dihydroxypropan-2-yl)-5-((1,3-dihydroxypropan-2-yl)carbamoyl)-1H-pyrrole-2-carboxylic acid;
- 1-(1,2-dihydroxypropan-3-yl)-5-((1,2-dihydroxy propan-3-yl)carbamoyl)-1H-pyrrole-2-carboxylic acid;
- 1-(2-hydroxyethyl)-5-((2-hydroxyethyl)carbamoyl)-1H-pyrrole-2-carboxylic acid;

- 1-benzyl-5-(benzylcarbamoyl)-1H-pyrrole-2-carboxylic acid;
- 1-octyl-5-(octylcarbamoyl)-1H-pyrrole-2-carboxylic acid;
- 1-(1-hydroxypropan-2-yl)-5-((1-hydroxypropan-2-yl)carbamoyl)-1H-pyrrole-2-carboxylic acid;
- 1-(2-mercaptoethyl)-5-((2-mercaptoethyl)carbamoyl)-1H-pyrrole-2-carboxylic acid;
- 1-(3-(triethoxysilyl)propyl)-5-((3-(triethoxysilyl)propyl)carbamoyl)-1H-pyrrole-2-carboxylic acid;
- 1-(carboxymethyl)-5-((carboxymethyl)carbamoyl)-1H-pyrrole-2-carboxylic acid; and
- 1-(2-aminoethyl)-5-((2-aminoethyl)carbamoyl)-1H-pyrrole-2-carboxylic acid.

Typically said compound of formula (I) is present in an amount of at least 0.1 phr, preferably of between 0.5 phr and 6 phr, even more preferably of between 0.5 phr and 4 phr.

Of note, the compounds of formula (I) are characterized by the presence of a carbonyl group in each ortho position to the pyrrole nitrogen atom. Without being bound to a specific theory, it is hypothesized that this group may allow for a greater and stronger interaction of the compounds within the elastomer composition, i.e. either with the one or more elastomers or the reinforcing filler(s) present.

The pyrrole derivatives (i.e. the compounds of formula (I)) used in the elastomer composition according to the present invention can be prepared according to methods known in the art.

For example, a convenient synthetic route for the compounds according to formula (I) and (II) above wherein groups R and R', or $R_{23}$-$R_{24}$, are the same, is that of reacting a suitable primary amine with 3-hydroxy-2-oxo-2H-pyran-6-carboxylic acid at a temperature of about 50-100°C until completion of the reaction. The pyrrole derivative of formula (I) is then isolated by column chromatography.

Of note, 3-hydroxy-2-oxo-2H-pyran-6-carboxylic acid can be easily and efficiently obtained from known biomolecules (e.g. from sugars) via processes in line with the requirements of green chemistry: the starting material for the synthesis of the compounds of formula (I) is therefore easily obtainable from inexpensive and bio-compatible sources.

The following compounds of formula (I) can all be obtained via the synthetic route disclosed above.

| Amine | Compound | |
|---|---|---|
| Isoserinol (3-amino-1,2-propanediol) | 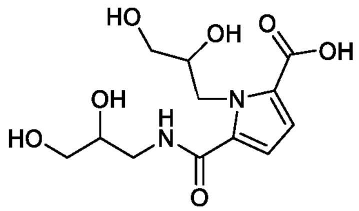 | 1-(1,2-dihydroxypropan-3-yl)-5-((1,2-dihydroxy propan-3-yl)carbamoyl)-1*H*-pyrrole-2-carboxylic acid |
| Serinol (2-amino-1,3-propanediol) | 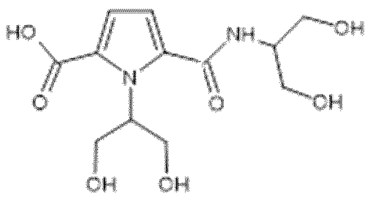 | 1-(1,3-dihydroxypropan-2-yl)-5-((1,3-dihydroxypropan-2-yl)carbamoyl)-1H-pyrrole-2-carboxylic acid |
| Ethanolamine | 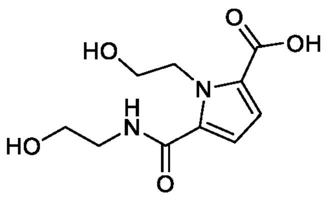 | 1-(2-hydroxyethyl)-5-((2-hydroxyethyl)carbamoyl)-1*H*-pyrrole-2-carboxylic acid |
| Benzylamine | 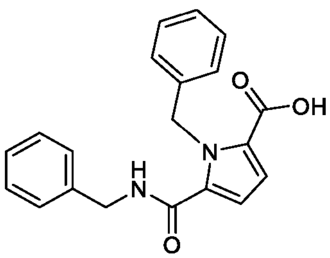 | 1-benzyl-5-(benzylcarbamoyl)-1*H*-pyrrole-2-carboxylic acid |

(continued)

| Amine | Compound | |
|---|---|---|
| Octylamine | 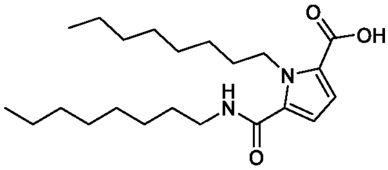 | 1-octyl-5-(octylcarbamoyl)-1*H*-pyrrole-2-carboxylic acid |
| Alaninol (3-amino-propan-1-ol) | 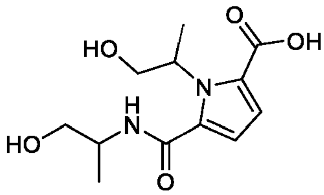 | 1-(1-hydroxypropan-2-yl)-5-((1-hydroxypropan-2-yl) carbamoyl)-1H-pyrrole-2-carboxylic acid |
| Cysteamine (2-amino ethanethiol) | 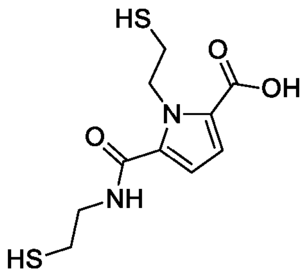 | 1-(2-mercaptoethyl)-5-((2-mercaptoethyl)carbamoyl)-1H-pyrrole-2-carboxylic acid |
| 3-(triethoxysilyl) propylamine | 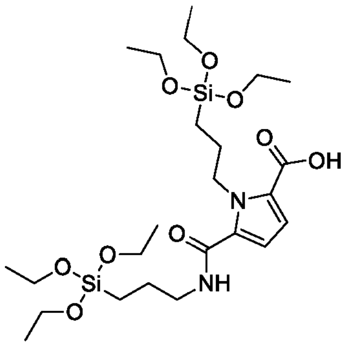 | 1-(3-(triethoxysilyl)propyl)-5-((3-(triethoxysilyl) propyl)carbamoyl)-1H-pyrrole-2-carboxylic acid |
| Glycine (2-amino acetic acid) | 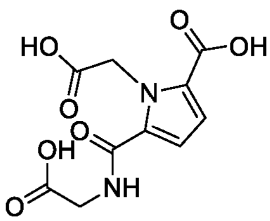 | 1-(carboxymethyl)-5-((carboxymethyl)carbamoyl )-1H-pyrrole-2-carboxylic acid |
| Ethylenediamine (1,2-diamino ethane) | 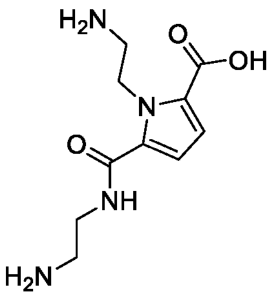 | 1-(2-aminoethyl)-5-((2-aminoethyl)carbamoyl)-1H-pyrrole-2-carboxylic acid |

As it will be understood by those of ordinary skill in the art, compounds of formula (I) can also be synthetized via synthetic routes different from that reported above, which will be selected by the expert in the field based on the common

general knowledge.

Preferably said at least one $sp^2$ hybridized carbon allotrope is selected from the group consisting of: carbon black, graphene, 2 layer graphene, from 3 to 10 layer graphene, graphite, high surface area graphite, single wall or multiwall carbon nanotubes, carbon nanotubes of longitudinal or helicoid extension, nanocones, nanohorns, nanotoroids, fullerene and mixtures thereof.

Even more preferably, said $sp^2$ hybridized carbon allotrope is selected from the group consisting of: carbon black, graphene, graphite, high surface area graphite, single wall or multiwall carbon nanotubes and mixtures thereof.

In a particularly preferred embodiment, the $sp^2$ hybridized carbon allotrope is carbon black.

Typically said at least one $sp^2$ hybridized carbon allotrope is present in an amount of at least 1 phr, preferably of between 2 phr and 70 phr, even more preferably of between 3 phr and 50 phr.

The elastomer composition according to the present invention typically further comprises additional reinforcing fillers selected from the group comprising: carbon black, silica, layered silicates, mixed oxides of aluminium and magnesium with lamellar structure, alumina and silico aluminates and mixtures thereof. Preferably, said additional reinforcing fillers are silica and/or carbon black.

In preferred embodiments of the invention, carbon black is present in an amount of at least 10 phr, preferably of from 15 phr to 70 phr.

In embodiments of the invention silica is present in an amount of from 0 to 100 phr, preferably of from 0 to 50 phr.

In an embodiment of the invention the elastomer composition comprises carbon black as reinforcing filler and at least one $sp^2$ hybridized carbon allotrope. In this embodiment the total amount of carbon black is of at least 15 phr, preferably from 30 phr to 70 phr.

In an embodiment of the present invention, the elastomer composition comprises an adduct between the compound of formula (I) as defined above and said at least one $sp^2$ hybridized carbon allotrope. That is to mean that the compound of formula (I) and the $sp^2$ hybridized carbon allotrope are provided as pre-formed adducts. Typically, said adduct is present in an amount of at least 1 phr, preferably of from 2 phr to 70 phr, even more preferably of from 9 phr to 50 phr.

Therefore in the elastomer composition according to the present invention, the total amount of reinforcing fillers, meaning the sum of the amount of the at least one $sp^2$ hybridized carbon allotrope, preferably provided as a pre-formed adduct with the compound of formula (I) as defined above, and further reinforcing fillers may reach from 11 to 150 phr, preferably from 20 to 150 phr, even more preferably from 30 to 80 phr

The elastomer compositions according to the present invention comprise at least one unsaturated elastomer which is preferably selected in the group consisting of: poly(1,4-cis-isoprene), either natural rubber or synthetic polymer, poly(3,4-isoprene), poly(butadiene) (in particular poly(butadiene) with a high content of 1,4-cis units, i.e. poly(1,4-cis-butadiene)), isoprene/isobutene copolymers, halogenated isoprene/isobutene copolymers such as for example halogenated butyl rubber, in particular chlorobutyl and bromobutyl rubber, 1,3-butadiene/acrylonitrile copolymers, styrene/1,3-butadiene copolymers, styrene/isoprene/1,3-butadiene copolymers, styrene/1,3-butadiene/acrylonitrile copolymers, and mixtures thereof.

The elastomer compositions according to the present invention can further contain at least one elastomer of one or more mono-olefins. The mono-olefins can be selected from: ethylene and 1-olefins with 3 to 12 carbon atoms, such as, for example, propylene, 1-butene, 1-pentene, 1-hexene, 1-octene, or mixtures of these mono-olefins.

The elastomer of one or more mono-olefins may comprise a diene, which generally has from 4 to 20 carbon atoms and is preferably selected from: 1,3-butadiene, isoprene, 1,4-hexadiene, 1,4-cyclohexadiene, 5-ethylidene-2-norbornene, 5-methylene-2-norbornene, vinylnorbornene or mixtures of these dienes. The diene can optionally be halogenated.

Among these elastomers of one or more mono-olefins, the following are preferred: ethylene/propylene copolymers (EPR) or ethylene/propylene/diene copolymers (EPDM) and poly(isobutene).

The elastomer compositions can further contain an unsaturated, diene or non-diene monomers-based elastomer, functionalized by reaction with a suitable terminating agent or coupling agents. In particular, the diene elastomer polymer can be obtained by anionic polymerization promoted by an organometallic initiator (in particular an alkyl-lithium) and terminated by reaction with suitable terminating agents or coupling agents such as, for example, epoxides, carbonyl compounds such as for example cyclohexanone and benzophenone, substituted or unsubstituted, imines, carbodiimides, alkyl-tin halides, alkoxysilanes or aryloxysilanes.

Advantageously, said elastomer composition contains a vulcanizing agent and the vulcanization takes place according to the known techniques, in particular with vulcanization systems based on sulphur commonly used for diene elastomer polymers. For this purpose, in the materials, after one or more steps of thermal-mechanical treatment, a vulcanizing agent based on sulphur is incorporated together with vulcanization accelerators. At the final treatment step, the temperature is generally maintained below 120°C and preferably below 100°C, so as to avoid any undesired pre-crosslinking phenomenon.

Preferably, the vulcanizing agent comprises vulcanization systems based on sulphur, comprising sulphur or molecules comprising sulphur (sulphur donors), together with vulcanization accelerators and/or activators known in the

art.

The activators which are particularly effective are compounds of zinc and in particular ZnO, $ZnCO_3$ and zinc salts of saturated or unsaturated fatty acids comprising from 8 to 18 carbon atoms, such as, for example, zinc stearate, which are preferably formed in situ in the elastomer composition from ZnO and fatty acid, or mixtures thereof.

The accelerators which are commonly used can be selected from: dithiocarbamates, guanidine, thiourea, thiazoles, sulfenamides, thiurams, amines and xanthates and mixtures thereof.

Preferred vulcanising agents are for example, stearic acid, ZnO, TBBS (N-tert-butyl-2-benzothiazyl sulfenamide) and sulphur or mixtures thereof.

According to a preferred embodiment, said elastomer composition contains a quantity of vulcanizing agent greater than or equal to about 1 phr, preferably greater than or equal to about 2 phr.

Preferably, the quantity of vulcanizing agent is less than or equal to about 20 phr, preferably less than or equal to about 10.

Advantageously the quantity of sulphur lies between about 0.5 phr and about 8 phr.

Said elastomer composition can contain other commonly used additives, selected on the basis of the specific application for which the composition is intended, for example: antioxidants, anti-ageing agents, plasticizers, adhesives, anti-ozone agents, modifying resins, or mixtures thereof.

In particular, for the purpose of improving workability, a plasticizer generally selected from mineral oils, plant oils, synthetic oils or mixtures thereof, such as, for example, aromatic oil, naphthenic oil, phthalates, soya oil or mixtures thereof can be added to said elastomer composition. The amount of plasticizer is generally from 0 phr to about 70 phr, preferably from about 5 phr to about 30 phr.

A further aspect of the present invention is a process for the preparation of the elastomer composition according to the invention, the process comprising the steps of:

a) providing one or more elastomers, at least one $sp^2$ hybridized carbon allotrope, a compound of formula (I) as defined above, optionally one or more additional reinforcing fillers, optionally one or more additives, and optionally one or more vulcanizing agents; and
b) performing at least one mixing step.

In an embodiment of the present invention, said compound of formula (I) and said at least one $sp^2$ hybridized carbon allotrope are provided as a pre-formed adduct.

The elastomer composition can be prepared by mixing together the polymeric components with the at least one $sp^2$ hybridized carbon allotrope and the compound of formula (I) according to the present invention, preferably provided as a pre-formed adduct, and together with the other reinforcing fillers and the other additives possibly present, according to techniques known in the art. The mixing can be performed, for example, using an open mixer of the "open-mill" type and/or an internal mixer of the type with tangential rotors (Banbury®), and/or with intermeshing rotors (Intermix™), and/or in continuous mixers of the Ko-Kneader™ type, and/or of the twin screw or multiscrew type and/or of the planetary type.

The components of the elastomer composition are not generally introduced all simultaneously into the mixer but are typically added in sequence. In particular, the vulcanization additives, such as the vulcanizing agents including possibly accelerants and retardants, are usually added in a downstream step relative to the incorporation and processing of all the other components.

In the vulcanizable or final vulcanized elastomer composition, the individual components of the elastomer composition do not always remain unaltered or are individually traceable in that they may be transformed, completely or in part, by effect of the interaction with other components, heat and/or mechanical processing.

A further aspect of the present invention is represented by a tyre for vehicle wheels comprising at least one structural element comprising a crosslinked elastomer material obtained by crosslinking of an elastomer composition according to the present invention.

In particular, said tyre comprises at least one carcass structure having opposite lateral edges associated with respective annular reinforcing structures, a belt structure applied in a position radially external relative to said carcass structure, a tread band applied in a position radially external to said carcass structure, and a pair of sidewalls applied laterally on opposite sides relative to said carcass structure.

Advantageously, said structural element is selected in the group consisting of tread band, sidewall, sidewall insert, layers of elastomer material radially internal relative to said tread band, for example, cushion and mini-sidewall, bead filler, and rubber coating of the textiles and the metals. In a particularly preferred embodiment, the structural element is the tread band.

Such structural elements can be advantageously prepared with the elastomer composition according to the present invention, characterized by low hysteresis and a limited Payne effect, thus obtaining a clear advantage on the road-holding and in general on the performance of the tyre, such as the resistance to the deformation due for example to an increase in the temperature. In particular, as disclosed above, the elastomer composition according to the present

invention is able to impart the desired mechanical-dynamical properties to the tyre with a limited, or null, use of silica, overcoming the disadvantages known in the art and discussed in the previous paragraphs.

The tyre according to the invention can be used on vehicles with two, three, four or even more wheels.

The tyre according to the invention can be for summer or winter use or for all seasons.

The tyre according to the present invention can be manufactured according to a process comprising:

- assembling components of a raw tyre on at least one building drum;
- moulding and vulcanizing the raw tyre;

wherein at least one component of the raw tyre contains the vulcanizable elastomer composition as previously described.

The term "raw" is generally used to indicate a material, a composition, a component or a tyre not yet vulcanized.

Figure 1 illustrates in radial half-section an exemplary tyre for vehicle wheels according to the invention.

In Figure 1, "a" indicates an axial direction and "X" indicates a radial direction, in particular X-X indicates the trace of the equatorial plane. For simplicity, Figure 1 shows only one portion of the tyre, the remaining portion not represented being identical and positioned symmetrically relative to the equatorial plane "X-X".

The tyre 100 for four-wheeled vehicles comprises at least one carcass structure, comprising at least one carcass layer 101 presenting mutually opposite terminal edges bonded to respective annular anchoring structures 102, called bead cores, possibly combined with a bead filler 104.

The zone of the tyre comprising the bead core 102 and the filler 104 forms a bead structure 103 intended for the anchoring of the tyre on a corresponding mounting rim, not illustrated.

The carcass structure is usually of the radial type, namely the reinforcing elements of at least one carcass layer 101 lie essentially on planes comprising the axis of rotation of the tyre and essentially perpendicular to the equatorial plane of the tyre. Said reinforcing elements are generally represented by textile cords, for example rayon, nylon or polyester (for example polyethylene naphthalate PEN). Each bead structure is connected to the carcass structure by folding behind opposite lateral edges of the at least one carcass layer 101 around the annular anchoring structure 102, so as to form the so-called carcass turnups 101a.

In one embodiment, the coupling between carcass structure and bead structure can be provided via a second carcass layer, not represented in Figure 1, applied in an axially external position relative to the first carcass layer.

An anti-abrasive strip 105 possibly implemented with an elastomer composition according to the invention is arranged in a position external to each bead structure 103.

To the carcass structure, a belt structure 106 comprising one or more belt layers 106a, 106b placed in radial superposition relative to one another and to the carcass layer, having typically textile or metal reinforcing cords incorporated within one layer of vulcanized elastomer material is associated.

Such reinforcing cords can have a crossed orientation relative to a circumferential development direction of the tyre 100. "Circumferential" direction is understood to mean a direction generally oriented in the direction of rotation of the tyre.

In a position radially more external to the belt layers 106a, 106b at least one reinforcing layer at zero degrees 106c can be applied, commonly known as "0° belt", which typically incorporates a plurality of elongated reinforcing elements, typically metal or textile cords, oriented in an essentially circumferential direction, thus forming an angle of a few degrees (for example an angle between about 0° and 6°) relative to a direction parallel to the equatorial plane of the tyre, and coated with vulcanized elastomer material.

In a position radially external to the belt structure 106, a tread band 109 in vulcanized elastomer material is applied.

Further, on the lateral surfaces of the carcass structure, each extending from one of the lateral edges of the tread 109 up to where it coincides with the respective bead structure 103, respective sidewalls 108 in vulcanized elastomer material are applied at an axially external position.

In a radially external position, the tread band 109 has a rolling surface 109a intended to make contact with the ground. Circumferential grooves, which are connected by transverse indentations (not shown in Figure 1) such as to define a plurality of small blocks of various forms and dimensions distributed on the rolling surface 109a, are generally implemented in this surface 109a, which for simplicity is shown smooth in Figure 1.

An underlayer 111 in vulcanized elastomer material can be positioned between the belt structure 106 and the tread band 109.

A strip of the elastomer composition 110, commonly known as "mini-sidewall", in vulcanized elastomer material can optionally be present in the connecting zone between the sidewalls 108 and the tread band 109, this mini-sidewall being generally obtained by co-extrusion with the tread band 109 and allowing an improvement in the mechanical interaction between the tread band 109 and the sidewalls 108. Preferably the end portion of the sidewall 108 directly covers the lateral edge of the tread band 109.

In the case of tyres with no inner tube, a layer of rubber 112, generally known as a "liner", which provides the

necessary impermeability to the air inflating the tyre, can also be provided in a radially internal position relative to the carcass structure 101.

The rigidity of the tyre 108 in the radial direction can be improved by endowing the bead structure 103 with a reinforcing layer 120 generally known as a "flipper" or strip additional insert.

The flipper 120 is a reinforcing layer which is wound around the respective bead core 102 and the bead filler 104 so as to at least partially surround them.

The flipper 120 typically comprises a plurality of textile cords, incorporated within a layer of vulcanized elastomer material.

The tyre bead structure 103 can contain a further protective layer which is generally known by the term "chafer" 121 or protective strip and which has the function of further increasing the rigidity and/or integrity of the bead structure 103.

The chafer 121 usually comprises a plurality of cords incorporated within a rubber coating layer of vulcanized elastomer material. Such cords are generally implemented in textile materials (for example aramid or rayon) or in metallic materials (for example steel cords).

A layer or sheet of elastomer material can be positioned between the belt structure and the carcass structure (not shown in Figure 1). The layer can have a uniform thickness. Alternatively, the layer can have a thickness variable in the axial direction. For example, the layer can have a maximum thickness close to its axially external edges relative to the central (crown) zone.

Advantageously the layer or sheet can extend over an area substantially corresponding to the development surface of said belt structure.

In a preferred embodiment, a layer or sheet of elastomer material as described above can be placed between the belt structure and the tread band, where said supplementary layer or sheet preferably extends over an area substantially corresponding to the development surface of the belt structure.

In still another aspect, the present invention relates to an adduct between a $sp^2$ hybridized carbon allotrope and a compound of formula (I)

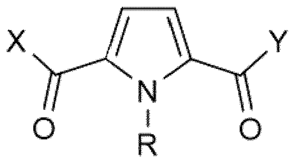

(I)

wherein X and Y are independently selected from the group consisting of:
-NHR' and OR"; and
wherein R" is selected from the group consisting of hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, and heteroaryl; and
wherein R and R' are independently selected from the group consisting of: hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, and carboxyl;
or R and/or R' is

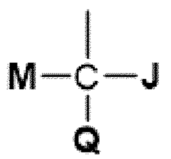

wherein M, Q and J are independently selected in a group consisting of amine, hydroxyl, hydrogen, linear or branched C1-C10 alkyl, and linear or branched C2-C10 alkenyl or alkynyl, or in a group consisting of:

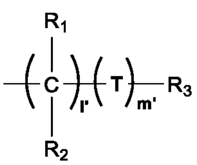 , 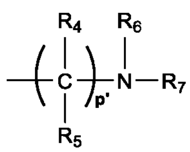 , 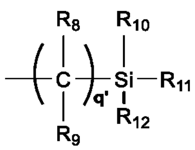 , 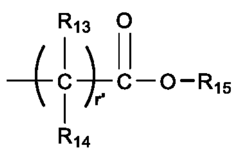 ,

wherein $R_1$-$R_{15}$ are independently selected from the group consisting of: hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, and carboxyl;
and wherein $R_{10}$-$R_{12}$ can be $-OCH_2-CH_3$, or $-OCH_3$;
and wherein $R_{14}$ can be $-CH_2-SH$, or $-CH_2-CH_2-S-CH_3$;
and wherein T is -O- or -S-, and when T is -S- then p' is an integer from 1 to 4, when T is -O- then p' is an integer from 1 to 2;
and wherein l', p', q', r' are independently integers from 0 to 12;
or R and/or R' is

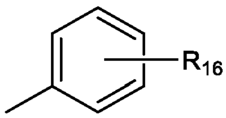
or
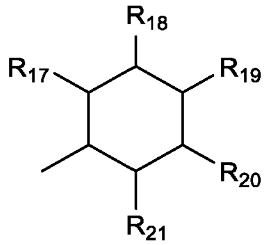
,

wherein $R_{16}$ is selected from the group consisting of hydrogen, alkyl, aryl, benzyl, amine, alkylamine, arylamine, benzylamine, or aminoaryl; and
$R_{17}$-$R_{21}$ are independently selected from the group consisting of: hydrogen, linear or branched C1-C10 alkyl, linear or branched C2-C10 alkenyl or alkynyl, and 1- (4-aminocyclohexyl) methylene.

In a preferred embodiment, said R and R' are the same.

Preferably, said R and R' are independently selected from the group consisting of hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, carboxyl, linear or branched C1-C10 alkyl-$NH_2$, linear or branched C1-C10 alkyl-SH, linear or branched C1-C10 alkyl-COOH, and linear or branched C1-C10 alkyl-$Si(OCH_2CH_3)_3$, wherein said alkyl groups are optionally substituted with one or more - OH groups. In a preferred embodiment, said R and R' are the same.

In a preferred embodiment, said compound of formula (I) is represented by formula (II) below

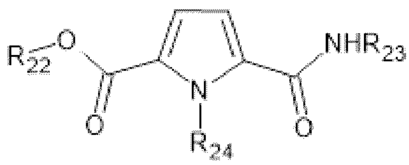
(II)

wherein said $R_{22}$ is selected from the group consisting of hydrogen and C1-C10 linear or branched alkyl group optionally substituted with one or more -OH groups; and said $R_{23}$-$R_{24}$ are the same and are selected from the group consisting of hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, linear or branched C1-C10 alkyl-$NH_2$, linear or branched C1-C10 alkyl-SH, linear or branched C1-C10 alkyl-COOH, and linear or branched C1-C10 alkyl-$Si(OCH_2CH_3)_3$, wherein said alkyl groups are optionally substituted with one or more -OH groups.

In particularly preferred embodiments of the present invention, said compound of formula (I) is selected from the group consisting of:

- 1-(1,3-dihydroxypropan-2-yl)-5-((1,3-dihydroxypropan-2-yl)carbamoyl)-1H-pyrrole-2-carboxylic acid;
- 1-(1,2-dihydroxypropan-3-yl)-5-((1,2-dihydroxy propan-3-yl)carbamoyl)-1H-pyrrole-2-carboxylic acid;
- 1-(2-hydroxyethyl)-5-((2-hydroxyethyl)carbamoyl)-1H-pyrrole-2-carboxylic acid;
- 1-benzyl-5-(benzylcarbamoyl)-1H-pyrrole-2-carboxylic acid;
- 1-octyl-5-(octylcarbamoyl)-1H-pyrrole-2-carboxylic acid;
- 1-(1-hydroxypropan-2-yl)-5-((1-hydroxypropan-2-yl)carbamoyl)-1H-pyrrole-2-carboxylic acid;
- 1-(2-mercaptoethyl)-5-((2-mercaptoethyl)carbamoyl)-1H-pyrrole-2-carboxylic acid;
- 1-(3-(triethoxysilyl)propyl)-5-((3-(triethoxysilyl)propyl)carbamoyl)-1H-pyrrole-2-carboxylic acid;
- 1-(carboxymethyl)-5-((carboxymethyl)carbamoyl)-1H-pyrrole-2-carboxylic acid; and

- 1-(2-aminoethyl)-5-((2-aminoethyl)carbamoyl)-1H-pyrrole-2-carboxylic acid.

Preferably the $sp^2$ hybridized carbon allotropes used to prepare the adducts according to the present invention are as defined above, i.e. are selected in the group consisting of carbon black, graphene (possibly graphene with 2 layers (*bi-layer graphene*) or graphene with few layers (from 3 to 10; *few layer graphene*)), graphite, in particular high surface area graphite, single wall or multiwall carbon nanotubes, carbon nanotubes with longitudinal or helicoid extension, nanocones, nanohorns, nanotoroids, fullerene, and mixtures thereof.

Even more preferably, said $sp^2$ hybridized carbon allotrope is selected from the group consisting of: carbon black, graphene, graphite, high surface area graphite, single wall or multiwall carbon nanotubes and mixtures thereof.

In a particularly preferred embodiment, the $sp^2$ hybridized carbon allotrope is carbon black.

In one embodiment the $sp^2$ hybridized carbon allotrope contains functional groups selected in the group comprising:

- oxygenated groups, preferably hydroxyls or epoxides;
- groups containing carbonyls, preferably aldehydes, ketones or carboxylic acids;
- groups containing nitrogen atoms, preferably amines, amides, nitriles, diazonium salts or imines;
- groups containing sulphur atoms, preferably sulphides, disulphides, mercaptans, sulfones, or sulfiinic and sulphonic groups.

In particularly preferred embodiments of the present invention, the adducts comprise a carbon allotrope selected from the group consisting of: carbon black, graphene, graphite, high surface area graphite, or single wall or multiwall carbon nanotubes, and a compound of formula (I) selected from:

- 1-(1,3-dihydroxypropan-2-yl)-5-((1,3-dihydroxypropan-2-yl)carbamoyl)-1*H*-pyrrole-2-carboxylic acid;
- 1-(1,2-dihydroxypropan-3-yl)-5-((1,2-dihydroxypropan-3-yl)carbamoyl)-1*H*-pyrrole-2-carboxylic acid;
- 1-(2-hydroxyethyl)-5-((2-hydroxyethyl)carbamoyl)-1*H*-pyrrole-2-carboxylic acid;
- 1-benzyl-5-(benzylcarbamoyl)-1*H*-pyrrole-2-carboxylic acid;
- 1-octyl-5-(octylcarbamoyl)-1*H*-pyrrole-2-carboxylic acid;
- 1-(1-hydroxypropan-2-yl)-5-((1-hydroxypropan-2-yl)carbamoyl)-1*H*-pyrrole-2-carboxylic acid;
- 1-(2-mercaptoethyl)-5-((2-mercaptoethyl)carbamoyl)-1H-pyrrole-2-carboxylic acid;
- 1-(3-(triethoxysilyl)propyl)-5-((3-(triethoxysilyl)propyl)carbamoyl)-1H-pyrrole-2-carboxylic acid;
- 1-(carboxymethyl)-5-((carboxymethyl)carbamoyl)-1H-pyrrole-2-carboxylic acid; and
- 1-(2-aminoethyl)-5-((2-aminoethyl)carbamoyl)-1H-pyrrole-2-carboxylic acid.

Preferably, the adducts of the present invention are characterized by a weight ratio between allotrope and pyrrole derivative comprised from 100:1 to 1:10, still more preferably from 100:1 to 1:1.

Due to the fact that they are more or less reactive aromatic systems, carbon allotropes can form various types of intermolecular interactions both of the covalent type and via more labile intermolecular interactions.

Without being bound by any theory, in the adduct formation reaction, the following two types of interaction can be hypothesized:

(i) $\pi$-$\pi$ interaction: an interaction which can exist between systems which possess $\pi$ electrons, thus having $sp^2$ or sp hybridization. The interaction is between a pair of $\pi$ electrons and a $\sigma$ orbital, or between the electrons of a $\sigma$ orbital and a $\pi$ orbital, or again between the electrons of two $\pi$ orbitals. This type of adduct is also known as a "$\pi$ complex" and is characterized by so-called *stacking,* i.e. a stacked arrangement of the aromatic molecules.

(ii) covalent bonds between the compound of formula (I) and the carbon allotrope, via reactions typical of polycyclic aromatic systems such as for example: 1,3-dipolar cycloadditions, Diels-Alder reaction.

It is believed that the presence of electron withdrawing groups, such as the carboxylic groups, enhances the reactivity of the pyrrole compounds with the $sp^2$ carbon allotropes by means of a Diels-Alder cycloaddition.

The adduct formation can also take place via the interaction between the functional groups of the pyrrole derivative of formula (I) and the functional groups possibly present on the carbon allotrope. These interactions can be of an intermolecular nature, such as hydrogen bonds and dipole interactions, or of a covalent nature such as for example an esterification via reaction with an acidic group.

The implementation of this interaction (whether of intermolecular or covalent nature) can also be effected solely by input of energy (for example mechanical or thermal energy, or by photon irradiation) without the use of solvents, thus according to a more "green" embodiment.

Accordingly, a further aspect of the present invention is a process for the preparation of an adduct between a $sp^2$

hybridized carbon allotrope and a compound of formula (I) as defined above, comprising the steps of:

i) providing a mixture of a compound of formula (I) as defined above and a $sp^2$ hybridized carbon allotrope; and
ii) supplying energy to the mixture obtained in step i) obtaining an adduct; and
iii) optionally separating the adduct obtained.

This process, particularly steps i)-iii), refers to the formation of the adduct prior to preparation of the elastomer composition, as a pre-formed adduct.

In an embodiment the mixture provided in step i) is a powder mixture.

In another embodiment, the process according to the present invention comprises between step i) and ii) a further step i') where said mixture is suspended in a solvent, preferably a non-polar solvent.

Preferably the solvent used in step i') is selected from: non-polar hydrocarbon solvents, such as decaline, hexane, cyclohexane, toluene.

Still in a further embodiment, the process according to the present invention comprises the following steps, to be performed prior to steps i)-iii), to provide the mixture of step i):

A. providing a compound of formula (I) and a $sp^2$ hybridized carbon allotrope in a polar protic or aprotic solvent selected in the group consisting of: water, alcohols, carbonyl solvents such as acetone, esters such as ethyl acetate, dimethyl sulfoxide, acetonitrile, ethers, and mixtures thereof;
B. forming a mixture, preferably via sonication or stirring;
C. removing said solvent from the mixture obtained in step B).

The process of solvent removal from the mixture obtained (step C) can occur by means of any suitable method for solvent removal, such as for example evaporation under vacuum, spray-drying, etc.

The energy transfer of step ii) of the process according to the present invention is performed in order to improve the interaction between the compound of formula (I) and the carbon allotrope. In the absence of energy transfer, this interaction would be weaker and could lead to the partial release of the compound of formula (I) from the carbon allotrope, in particular if the adduct is in an environment of a polar nature. Preferably, said energy is supplied in step ii) for a time period of at least 1 minute, preferably of at least 5 minutes.

The energy forms which can be transferred to the composition to contribute to the adduct formation are: mechanical energy, thermal energy, and energy by irradiation with photons, or a combination of two or more of these energy forms.

*Mechanical energy*

The mechanical treatment can for example consist in placing the powder obtained (allotrope/compound of formula (I)) in a ball mill, for example comprising a jar equipped with stainless steel spheres. Once closed, the jar is placed in a planetary mixer and is left to rotate at a rate from 200 to 500 rpm for periods from 1 to 360 minutes. Immediately afterwards, the powder is transferred.

The mechanical treatment is used both to promote the separation of the carbon allotropes into their fundamental components (for example exfoliation in the case of graphite), and to favour the relative dispersion of the allotrope and of the compound of formula (I), in order to obtain a better distribution of the compound of formula (I) on the allotrope, and to induce the formation of a more stable interaction.

*Thermal energy*

The thermal treatment can for example consist in placing the powder obtained (carbon allotrope/compound of formula (I)) in a reaction flask equipped with a condenser, or in a closed vessel. Once the reactor has been placed on a heating plate, the reaction is carried out at temperatures of from 25 to 180°C. The heating is maintained for a time from 15 and 360 minutes.

Or the thermal treatment can be carried out via a fluidized bed reactor or via spray-drying.

*Photons*

The photon treatment can for example consist in placing the powder obtained (carbon allotrope/compound of formula (I)) in a laboratory crystallizer forming a thin layer or in placing the powder in a closed quartz vial. Once the reactor has been set up within a closed chamber equipped with a 254 nm low-pressure mercury, lamp (or using a Rayonet® reactor equipped with the same type of reactor) the mixture is irradiated for times variable from 30 to 180 minutes. At the end of this

time, the mixture is transferred and analysed.

Preferably the thermal energy is supplied at a temperature of from 50°C to 180°C, for example for a time of from 5 to 360 minutes.

Preferably the mechanical energy is supplied for a time of from 1 to 360 minutes.

Preferably the energy for irradiation with photons is supplied at a wavelength of from 200 to 380 nm, for example for a time of from 30 to 180 minutes.

The process according to this embodiment makes it possible to obtain a homogeneous dispersion of the carbon allotrope and of at least one compound of formula (1) and thus a homogeneous dispersion of the compound of formula (1) in the carbon-based filler.

The adducts obtained according to the present invention may be used as reinforcing fillers for the preparation of different mixtures either elastomeric or of composite material.

The present invention will be further illustrated hereinafter by means of experimental examples, which are provided for purely illustrative purposes and without any limitation of this invention.

## EXPERIMENTAL PART

*Materials*

All the reagents and solvents for the preparation of the pyrrole derivatives were obtained from Sigma-Aldrich and used without further purification.

The $^1$H-NMR spectra were recorded on samples dissolved in deuterated chloroform ($CDCl_3$) and dimethyl sulfoxide (DMSO-$d_6$).

The elastomers used are:

- poly(1,4-*cis*-isoprene) (NR), solid natural rubber SIR20, from Eatern GR Thailand - Chonburi. Mooney viscosity (ML(1+4) @ 100 °C): 73 MU;
- poly(1,4-*cis*-butadiene) (butadiene rubber, BR) Europrene Neocis® from - Polimeri Europa

The carbon allotrope used is:

- Carbon black N326 from Cabot Corporation

Other ingredients for the preparation of the elastomer compositions:

- X50S (bis(triethoxysilylpropyl) polysulfide), from Evonik
- Silica grade Zeosil® 1165 MP from Solvay
- Stearic acid from Undesa
- ZnO from Zincol Ossidi;
- 6PPD ((1,3-dimethylbutyl)-*N*'-phenyl-p-phenylenediamine) from Eastman;
- TBBS (N-tert-butyl-2-benzothiazyl sulphenamide) from Solutia Eastman
- Sulphur from International Sulphur Inc
- PVI (pre-vulcanization inhibitor, N-(Cyclohexylthio)phthalimide) from Brenntag Spa,

*Characterization*

The pyrrole compounds obtained were analysed by NMR spectroscopy. The $^1$H-NMR and $^{13}$C-NMR spectra were recorded with a Bruker 400 MHz (100 MHz $^{13}$C) instrument operating at 298 K. The chemical shifts are stated in parts per million (ppm) with the solvent residues peaks as internal standard (DMSO-$d_6$: $\delta$ H= 2.50 ppm, $CDCl_3$: $\delta$ H= 7.26 ppm).

The adducts between carbon allotropes and the pyrrole compounds were characterized as follows.

Thermogravimetric analysis (TGA) is a quantitative analytical technique which provides the weight loss of the material analysed as a function of time and temperature. In other words, the material is heated with a given heating regime and can undergo transformations which lead to the loss of part or all of the material itself, which passes into the vapour phase. The TGA under a current of $N_2$ (60 mL/min) was performed using a Mettler TGA SDTA/851 instrument in accordance with the standard method ISO9924-1. The samples (10 mg) were heated from 30 to 300°C at 10°C/min, maintaining at 300°C for 10 min, and immediately afterwards heating to 550°C (20°C/min). After having maintained them 550°C for 15 min, further heating to 650°C follows at a rate of 30°C/min and maintaining at 650°C for 20 min under a current of air (60 mL/min).

The functionalization yield of the adducts obtained, i.e. the percentage quantity by weight of molecule bound to

the sp$^2$ hybridized carbon allotrope, was calculated using the following equation:

$$\textit{Functionalization yield (\%)} = 100 * \frac{\text{compound of formula (1) mass\% in adduct after washing}}{\text{compound of formula (1) mass\% in adduct before washing}}$$

The values stated for the purposes of the calculation make reference to the quantity of pyrrole compound, contained in the adducts, determined by TGA before and after the purification procedure and make reference to the % weight losses determined from the TGA graph in the temperature range which runs from 0 to 900°C. Figures 2a-2b show an exemplary TGA spectrum, respectively before and after the purification procedure, of an adduct according to the invention.

The elastomer compositions were vulcanized and characterized by the following procedures and techniques.

The tensile tests were performed on test pieces of the dumbbell ISO 3 type with rectilinear axis. Dumbell samples were cut out of 100x100x2 mm rubber sheets obtained curing the green compound in a mould at 170°C for 10 minutes in a hydraulic press. The static mechanical properties were measured at 23°C in accordance with the standard ISO 37:2005. In particular, the load was measured at different levels of elongation, 50%, 100% and 300%, named in sequence $\sigma$100, $\sigma$200, $\sigma$300, and the stress at break and the elongation at break named in sequence $\sigma$B and $\varepsilon$B.

The dynamic mechanical properties were measured by applying a dynamic stress via shear stress, at constant frequency and at constant temperature, increasing the deformation amplitude. The test was performed with a Monsanto RPA 2000 rheometer. The samples of elastomer material composition were held in the rheometer at 50°C for 90 seconds, the stress was then applied at 50°C in the deformation amplitude range between 0.1% and 25%, with a frequency of 1 Hz, increasing the deformation amplitude in the range stated above. This treatment is performed to eliminate the "previous thermo-mechanical history". The vulcanization was then performed at 170°C for 20 minutes, with a frequency of 1.667 Hz and an angle of 6.98% (0.5 rad). The vulcanized sample was left in the instrument for 10 minutes at 50°C. The sinusoidal stress was then applied with the same conditions already stated, at 50°C. The sinusoidal stress is then again applied, always with the same experimental conditions. Curves are then obtained which state the value of the moduli as a function of the deformation amplitude. Such moduli are illustrated below. The G' modulus is the elastic modulus. The G" modulus is the viscous modulus. The ratio G"/G' is stated as Tan Delta. From the strain sweep test, the values of the following parameters are obtained: G'$\gamma$=0.28% which is the value of G' at minimum deformation, $\Delta$G' which is the difference between the value of G' at minimum deformation and the value of G' measured at the maximum deformation reached, G"max which is the maximum value of G" observed in the G" curve, and (tan delta)max which is the maximum value of tan delta observed in the curve.

The dynamic mechanical properties were further measured by application of an axial stress using an Instron dynamic device in compression-traction modality in accordance with the following methods. A sample of the green elastomer compositions vulcanized at 170°C for 10 minutes having a cylindrical shape (length = 25 mm; diameter = 14 mm), compression-preload up to 25% of the longitudinal deformation relative to the initial length and maintained at the predetermined temperature (equal to 10°C, +23°C and +70°C) for the entire time length of the test, was subjected to a sinusoidal dynamic tension having an amplitude of $\pm$3.5% relative to the length under preload, with a frequency of 10 Hz. The dynamic mechanical properties are expressed in terms of elastic dynamic modulus (E') and tan delta (dissipation factor) values. The value of Tan delta was calculated as the ratio between the viscous modulus (E") and the elastic modulus (E').

The static and dynamic mechanical properties measured are summarized in the following table 1.

Table 1

| **Static mechanical properties** | |
|---|---|
| **Symbol** | **Meaning** |
| $\sigma_{100}$ | Stress at 100% elongation |
| $\sigma_{200}$ | Stress at 200% elongation |
| $\sigma_{300}$ | Stress at 300% elongation |
| $\sigma_B$ | Stress at break |
| $\varepsilon_B$ | Elongation at break |
| **Dynamic mechanical properties measured by means of shear stress** | |
| **Symbol** | **Meaning** |
| **G'** | Storage (elastic) modulus |
| **$\Delta$G'** | G'$\gamma$ min - G'$\gamma$ max |

(continued)

| | |
|---|---|
| $\varepsilon_B$ | Elongation at break |
| **Dynamic mechanical properties measured by means of shear stress** | |
| **Symbol** | **Meaning** |
| **(tan delta)** $_{max}$ | maximum value of tan delta |
| **Dynamic mechanical properties measured by means of axial stress** | |
| **Symbol** | **Meaning** |
| E' | Storage modulus |
| **E"** | Loss (viscous) modulus |
| **tan delta** | E"/E' |

Example 1: Synthesis of 1-(1,3-dihydroxypropan-2-yl)-5-((1,3-dihydroxypropan-2-yl)carbamoyl)-1*H*-pyrrole-2-carboxylic acid

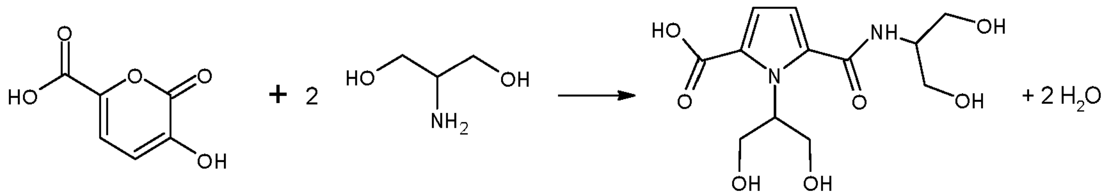

3-Hydroxy-2-oxo-2*H*-pyran-6-carboxylic acid (135 mg, 0.87 mmol) and serinol (2-amino-1,3-propanediol, 455 mg, 4.87 mmol) were loaded into a 25 ml round bottomed flask. The heterogeneous system was heated at 60°C, then closed and maintained under stirring at this temperature until completion of the reaction as evidenced by $^1$H-NMR and $^{13}$C-NMR analysis of a sample of the reaction mixture. 1-(1,3-dihydroxypropan-2-yl)-5-((1,3-dihydroxypropan-2-yl)carbamoyl)-1*H*-pyrrole-2-carboxylic acid (compound 1) was then isolated by column chromatography (75% yield).

*Examples 2-5: preparation of adducts between pyrrole derivatives and carbon black (CB)*

Example 2 - Preparation of the physical mixture of CB and compound (1)

In a 25 mL round bottomed flask were put in sequence CB N326 (1 g), compound 1 (0.1 g), obtained as disclosed in example 1 above, and acetone (10 ml).
The so obtained instable suspension was sonicated for 15 min, using a 2 L ultrasonic bath (260 W).
After this time, the solvent was removed by means of rotavapor to give a compound (1)/CB powder mixture (1 g).

Example 3 - Neat preparation of the adduct between compound (1) and CB

The compound (1)/CB physical mixture, prepared as described in Example 2, was stirred at 170°C, for 10 minutes, in the presence of air. Then, it was cooled to room temperature, and it was repeatedly washed with acetone. The final powder was obtained by filtration.
The functionalization yield, determined via TGA as disclosed above, was 89%.

Example 4 - Preparation of the adduct between compound (1) and CB in a solvent

The compound (1)/CB physical mixture, prepared as described in Example 2, was poured in a 25 ml double neck flask, under nitrogen flow, in presence of decaline (5 ml) as solvent.
The mixture was first stirred at 150°C for 5 minutes and then at 170°C per 10 minutes. Afterward, the mixture was cooled to room temperature, and it was repeatedly washed with acetone. The final powder was obtained by filtration.
The functionalization yield, determined via TGA as disclosed above, was 93%.

Example 5 - Preparation of the adduct between compound (1) and CB with long reaction time

In a 100 mL round bottom flask, CB (15.0 g), acetone (25 mL) and a solution of compound (1) (1.5 g) in acetone/water (5 mL) were sonicated for 15 min, using a 2 L ultrasonic bath (260 W). After this time, the solvent was eliminated by means of rotavapor to give a black powder, which was kept for 2 hours, under vigorous stirring, at 150°C.
The mixture was then cooled to room temperature and repeatedly washed with a mixture acetone/water (5/1 wt/wt) (3 × 20 mL). The final powder was obtained by filtration.
The functionalization yield, determined via TGA as disclosed above, was 84%.

*Preparation of elastomer compositions - General Description*

Elastomer compositions were prepared via melt blending, according to the compositions summarized in Table 3. The compound (1)/CB adduct used was the one obtained as disclosed in example 5 above.

Table 3

| . | **Ex. 6^** | **Ex. 7** | **Ex. 8** | **Ex. 9** | **Ex. 10** |
|---|---|---|---|---|---|
| | [phr] | [phr] | [phr] | [phr] | [phr] |
| NR (SIR-20) | 70 | 70 | 70 | 70 | 70 |
| BR | 30 | 30 | 30 | 30 | 30 |
| X50S | 5,6 | 5,6 | 5,6 | 5,6 | 5,6 |
| N326 | 30 | 30 | 30 | 30 | 30 |
| SILICA | 35 | 23,45 | 12 | 0 | 12 |
| ZnO | 4 | 4 | 4 | 4 | 4 |
| stearic acid | 2 | 2 | 2 | 2 | 2 |
| 6PPD | 2 | 2 | 2 | 2 | 2 |
| Sulphur | 2 | 2 | 2 | 2 | 2 |
| PVI | 0.5 | 0.5 | 0.5 | 0.5 | 0.5 |
| TBBS | 1.8 | 1.8 | 1.8 | 1.8 | 1.8 |
| N326/compound (1) | 0 | 9,9 | 19,72 | 30 | 0 |
| N326 | 0 | 0 | 0 | 0 | 18,73 |
| Compound (1) | 0 | 0 | 0 | 0 | 0,99 |
| ^comparison | | | | | |

Example 6 - Elastomer composition comprising silica and CB (comparative example)

70 phr of Natural Rubber (SIR-20) and 30 phr of poly(1,4-butadiene) (BR) were fed into a Brabender® internal mixer and masticated at 130°C for 1 minute. CB, Silica Zeosil 1156MP and X50S were added into the mixer and mixed for 3 minutes at the same temperature. ZnO, stearic acid and 6PPD were then added and mixed for 2 minutes.
The so obtained compound was unloaded at 130°C.
These composites were fed again into the internal mixer at 45°C. Sulphur, PVI and TBBS (N-tert-butyl-2-benzothiazyl sulfenamide) were then added, mixing for a further 2 minutes.
The final rubber composite was discharged and cooled at room temperature and, before the characterization, mixed few times in 2 rolls open mill.

Example 7 - Elastomer composition comprising silica, CB, and compound (1)/CB adduct

The procedure disclosed in Example 6 was repeated, however 33% of the silica was replaced with the compound (1)/CB adduct obtained in example 5. The same volume % of the filler was maintained.

Example 8 - Elastomer composition comprising silica, CB, and compound (1)/CB adduct

The procedure disclosed in Example 6 was repeated, however 66% of the silica was replaced with the compound (1)/CB adduct obtained in example 5. The same volume % of the filler was maintained.

Example 9 - Elastomer composition comprising CB and compound (1)/CB adduct

The procedure disclosed in Example 6 was repeated, however the silica was completely replaced with the compound (1)/CB adduct obtained in example 5. The same volume % of the filler was maintained.

Example 10 - Elastomer composition comprising silica, CB, and compound (1) as an ingredient

The procedure disclosed in Example 6 was repeated, however 66% of the silica was replaced with pristine CB, and 0,99 phr of compound (1), obtained as disclosed in Example 1, were added. The same volume % of the filler was maintained.

*Dynamic mechanical characterization of the elastomer compositions by means of shear stress*

The dynamic mechanical characterization was performed by applying a sinusoidal stress by means of shear stress, in accordance with the previously described operative modalities.

In Table 4, the data relating to the dynamic modulus G' at minimum deformation (0.1), to the variation of the G' modulus (ΔG') between 0.28% and 25% as deformation amplitude, to maximum value of the dissipative modulus G", and to the maximum value of tan delta are shown.

The curves of the variation in G' and of tan delta relative to the deformation amplitude are shown respectively in Figure 3 and in Figure 4.

Table 4

| | **Ex. 6^** | **Ex. 7** | **Ex. 8** | **Ex. 9** | **Ex. 10** |
|---|---|---|---|---|---|
| $G'_{MIN}$ | 0,67 | 0,65 | 0,53 | 0,46 | 0,59 |
| $G'_{MAX}$ | 0,94 | 0,94 | 0,81 | 0,66 | 0,77 |
| ΔG' | 1,40 | 1,44 | 1,52 | 1,42 | 1,31 |
| Tan Delta Max | 0,12 | 0,11 | 0,12 | 0,10 | 0,12 |
| ^comparison | | | | | |

The use of a pyrrole derivative (compound (1)), either as modifier of CB or as ingredient of the elastomer composition, allows the partial or total replacement of silica with CB, substantially reproducing the same dynamic-mechanical properties measured for the composition based on pristine CB. Indeed, the values of ΔG' and Tan Delta max of Examples 7-10 in Table 4 are absolutely in line with the values of the comparative composition (Ex. 6^).

ΔG' indicates the decrease of the elastic G' modulus as the strain amplitude increases, so it indicates the non-linearity of the modulus. This phenomenon is known as Payne effect and indicates the presence of a filler network, due to the supramolecular interaction of filler particles, either direct or mediated by the rubber chains. The disruption of the filler network is a source of dissipation of energy.

The Tan delta max is the parameter of election to indicate the dissipation of energy. Hence, the higher are the ΔG' and the Tan delta max values, the higher is the dissipation of energy.

To replace silica with CB and to have substantially the same values of ΔG' and Tan Delta max indicates that the use of the pyrrole derivative (compound (1)) together with CB, either in the form of an adduct or by adding each species separately in the elastomer composition, is indeed able to replace silica.

In particular, it is worth observing that the use of the pyrrole derivative (compound (1)) as an ingredient of the elastomer composition leads to the same Tan Delta value and to appreciably lower value of ΔG' (Table 4).

*Dynamic mechanical characterization of the elastomer compositions by means of axial stress*

In Table 5, the data obtained from the dynamic mechanical tests, performed by application of a sinusoidal axial stress, are shown. The experimental conditions for performing the tests have been stated above.

Table 5

| | Ex. 6^ | Ex. 7 | Ex. 8 | Ex. 9 | Ex. 10 |
|---|---|---|---|---|---|
| **E'@10°C** | 3,88 | 3,86 | 3,65 | 3,78 | 3,80 |
| **E"@10°C** | 0,66 | 0,62 | 0,60 | 0,64 | 0,67 |
| **TanDelta@10°C** | 0,17 | 0,16 | 0,16 | 0,17 | 0,18 |
| **E'@23°C** | 3,69 | 3,66 | 3,44 | 3,51 | 3,54 |
| **E"@23°C** | 0,55 | 0,51 | 0,49 | 0,52 | 0,56 |
| **TanDelta@23°C** | 0,15 | 0,14 | 0,14 | 0,15 | 0,16 |
| **E'@70°C** | 3,32 | 3,40 | 3,16 | 3,26 | 3,20 |
| **E"@70°C** | 0,46 | 0,42 | 0,41 | 0,44 | 0,48 |
| **TanDelta@70°C** | 0,14 | 0,12 | 0,13 | 0,13 | 0,15 |
| **-ΔE' ($E'_{10°C}$ - $E'_{70°C}$)** | 0,56 | 0,46 | 0,49 | 0,52 | 0,60 |
| ^comparison | | | | | |

The same comment reported above for the dynamic-mechanical characterization in the shear mode can be reported also for the results from the axial tests. The use of the pyrrole derivative (compound (1)), either as modifier of CB or as ingredient of the elastomer composition, allows the partial or total replacement of silica with CB, substantially reproducing the same dynamic-mechanical properties measured for the composition based on pristine CB (Ex. 6^).

Moreover, it is important to observe that:

(i) the values of the storage modulus E' are lower at low temperature for the compositions according to the invention. This means that the composition has lower rigidity at low temperature, and this is definitely an advantage;
(ii) the values of ΔE' (E'10°C - E'70°C) are lower or in line in case of ex. 10 for the compositions according to the invention. This means that the compositions have higher stability of the dynamic rigidity as the temperature increases;
(iii) the values of tan delta are in line at all the temperatures and are lower for the compositions with the compound (1)/CB adduct (Ex. 7-9), in particular when 66% of silica is replace with the adduct (Ex. 8). This is a clear advantage for an elastomer composition intended, for example, for internal compounds in tyres.

*Characterization of the elastomer compositions by means of tensile tests*

Tensile properties were determined by quasi static measurements as disclosed above. In Table 6, the data obtained from the tensile tests are shown.

Table 6

| | Ex. 6^ | Ex. 7 | Ex. 8 | Ex. 9 | Ex. 10 |
|---|---|---|---|---|---|
| **$\sigma_{100}$ (Mpa)** | 0,76 | 0,83 | 0,85 | 0,91 | 0,78 |
| **$\sigma_{200}$ (MPa)** | 1,08 | 1,21 | 1,28 | 1,37 | 1,11 |
| **$\sigma_{300}$ (MPa)** | 4,09 | 4,89 | 4,99 | 4,86 | 4,10 |
| **$\sigma_B$ (MPa)** | 22,36 | 25,13 | 20,29 | 19,88 | 24,19 |
| **$\varepsilon_B$ (%)** | 692,01 | 678,74 | 612,46 | 631,91 | 727,35 |
| **Energy J/cm$^3$** | 52,80 | 57,81 | 43,09 | 44,20 | 58,65 |
| ^comparison | | | | | |

The tensile properties confirm what already reported commenting the data from dynamic-mechanical characterization. The elastomer compositions of the invention have tensile properties absolutely in line with those of the comparative composition based on pristine CB (Ex. 6^).

In particular, it is worth observing that:

(i) the composition comprising the pyrrole derivative (compound (1), Ex. 10) as an ingredient has better overall ultimate properties

(ii) the composition wherein 33% silica has been replaced with the compound (1)/CB adduct (Ex. 7) has higher tensile strength, with similar elongation at break.

## Claims

1. An elastomer composition comprising at least one $sp^2$ hybridized carbon allotrope and a compound of formula (I)

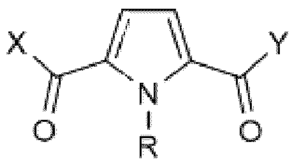

(I)

wherein X and Y are independently selected from the group consisting of:

-NHR' and OR", and

wherein R" is selected from the group consisting of hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, and heteroaryl; and

wherein R and R' are independently selected from the group consisting of: hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, and carboxyl;

or R and/or R' is

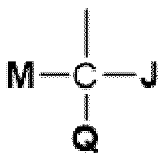

wherein M, Q and J are independently selected in a group consisting of amine, hydroxyl, hydrogen, linear or branched C1-C10 alkyl, and linear or branched C2-C10 alkenyl or alkynyl, or in a group consisting of:

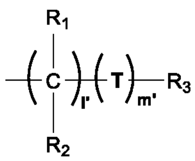 , 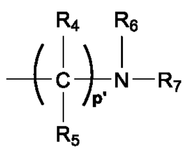 , 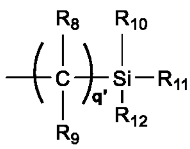 , 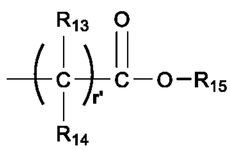 ,

wherein $R_1$-$R_{15}$ are independently selected from the group consisting of: hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, and carboxyl;

and wherein $R_{10}$-$R_{12}$ can be $-OCH_2-CH_3$, or $-OCH_3$;

and wherein $R_{14}$ can be $-CH_2-SH$, or $-CH_2-CH_2-S-CH_3$;

and wherein T is -O- or -S-, and when T is -S- then p' is an integer from 1 to 4, when T is -O- then p' is an integer from 1 to 2;

and wherein l', p', q', r' are independently integers from 0 to 12;

or R and/or R' is

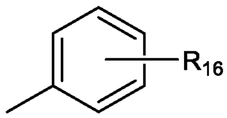 or 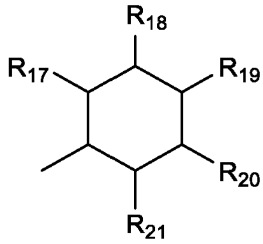 ,

wherein $R_{16}$ is selected from the group consisting of hydrogen, alkyl, aryl, benzyl, amine, alkylamine, arylamine, benzylamine, and aminoaryl; and

$R_{17}$-$R_{21}$ are independently selected from the group consisting of: hydrogen, linear or branched C1-C10 alkyl, linear or branched C2-C10 alkenyl or alkynyl, and 1- (4-aminocyclohexyl) methylene.

**2.** The elastomer composition according to claim 1, wherein R and R' are the same.

**3.** The elastomer composition according to claim 1, wherein said compound of formula (I) is represented by formula (II) below

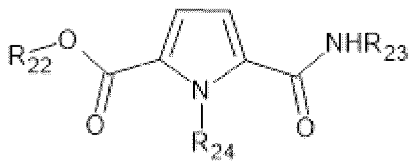

(II)

wherein said $R_{22}$ is selected from the group consisting of hydrogen and C1-C10 linear or branched alkyl group optionally substituted with one or more -OH groups; and said $R_{23}$-$R_{24}$ are the same and are selected from the group consisting of hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, linear or branched C1-C10 alkyl-$NH_2$, linear or branched C1-C10 alkyl-SH, linear or branched C1-C10 alkyl-COOH, and linear or branched C1-C10 alkyl-$Si(OCH_2CH_3)_3$, wherein said alkyl groups are optionally substituted with one or more -OH groups.

**4.** The elastomer composition according to any one of claims 1-3, wherein said $sp^2$ hybridized carbon allotrope is selected from the group consisting of: carbon black, graphene, 2 layer graphene, from 3 to 10 layer graphene, graphite, high surface area graphite, single wall or multiwall carbon nanotubes, carbon nanotubes of longitudinal or helicoid extension, nanocones, nanohorns, nanotoroids, fullerene and mixtures thereof.

**5.** The elastomer composition according to any one of claims 1-4, comprising at least one unsaturated elastomer selected in the group consisting of: poly(1,4-cis-isoprene), natural rubber or synthetic polymer, poly(3,4-isoprene), poly(butadiene), poly(butadiene) with a high content of 1,4-cis units, isoprene/isobutene copolymers, halogenated isoprene/isobutene copolymers, halogenated butyl rubber, chlorobutyl rubber, bromobutyl rubber, 1,3-butadiene/acrylonitrile copolymers, styrene/1,3-butadiene copolymers, styrene/isoprene/1,3-butadiene copolymers, and styrene/1,3-butadiene/acrylonitrile copolymers, and mixtures thereof.

**6.** The elastomer composition according to claim 5, further comprising at least one elastomer of one or more monoolefins, optionally containing a diene.

**7.** The elastomer composition according to any one of claims 1-6 further comprising additional reinforcing fillers selected from the group comprising: carbon black, silica, layered silicates, mixed oxides of aluminium and magnesium with lamellar structure, alumina and silico aluminates and mixtures thereof.

**8.** The elastomer composition according to claim 7, wherein said additional reinforcing fillers are selected in the group consisting of: silica, carbon black and mixtures thereof.

**9.** The elastomer composition according to any one of claims 1-8, further comprising one or more vulcanizing agents, optionally comprising an activator and/or an accelerator, and one or more additives selected from antioxidants, anti-ageing agents, plasticizers, adhesives, anti-ozone agents, modifying resins, and mixtures thereof.

**10.** Process for the preparation of an elastomer composition according to any one of claims 1-9, comprising the steps of:

a) providing one or more elastomers, at least one $sp^2$ hybridized carbon allotrope, a compound of formula (I) as defined in any one of claims 1-3, optionally one or more additional reinforcing fillers, optionally one or more additives, and optionally one or more vulcanizing agents; and
b) performing at least one mixing step.

**11.** The process according to claim 10, wherein said compound of formula (I) and said at least one $sp^2$ hybridized carbon allotrope are provided as a pre-formed adduct.

**12.** Tyre for vehicle wheels comprising at least one structural element comprising a crosslinked elastomer material obtained by crosslinking of an elastomer composition according to any one of claims 1-9.

**13.** Tyre according to claim 12, wherein said structural element is selected in the group consisting of: tread band, sidewall, sidewall insert, layers of elastomer material radially internal relative to said tread band, wherein said radially internal layers are preferably selected from: cushion and mini-sidewall, bead structures, and rubber coating of the textiles and the metals.

**14.** Adduct between a $sp^2$ hybridized carbon allotrope and a compound of formula (I)

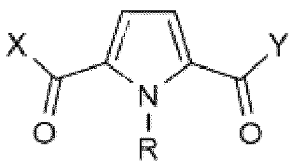

(I)

wherein X and Y are independently selected from the group consisting of:
-NHR' and OR", and
wherein R" is selected from the group consisting of hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, and heteroaryl; and
wherein R and R' are independently selected from the group consisting of: hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, and carboxyl;
or R and/or R' is

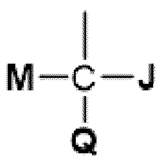

wherein M, Q and J are independently selected in a group consisting of amine, hydroxyl, hydrogen, linear or branched C1-C10 alkyl, and linear or branched C2-C10 alkenyl or alkynyl, or in a group consisting of:

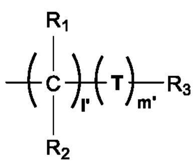 , 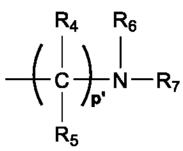 , 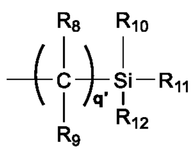 , 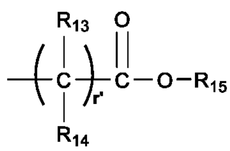 ,

wherein $R_1$-$R_{15}$ are independently selected from the group consisting of: hydrogen, linear or branched C1-C10 alkyl, linear or branched C2-C10 alkenyl or alkynyl, aryl, linear or branched C1-C10 alkyl-aryl, linear or branched

C2-C10 alkenyl-aryl, linear or branched C2-C10 alkynyl-aryl, heteroaryl, and carboxyl;
and wherein $R_{10}$-$R_{12}$ can be $-OCH_2-CH_3$, or $-OCH_3$;
and wherein $R_{14}$ can be $-CH_2-SH$, or $-CH_2-CH_2-S-CH_3$;
and wherein T is -O- or -S-, and when T is -S- then p' is an integer from 1 to 4, when T is -O- then p' is an integer from 1 to 2;
and wherein l', p', q', r' are independently integers from 0 to 12;
or R and/or R' is

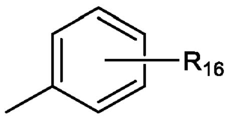
or
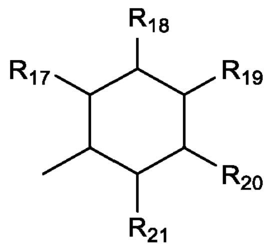
,

wherein $R_{16}$ is selected from the group consisting of hydrogen, alkyl, aryl, benzyl, amine, alkylamine, arylamine, benzylamine, and aminoaryl; and
$R_{17}$-$R_{21}$ are independently selected from the group consisting of: hydrogen, linear or branched C1-C10 alkyl, linear or branched C2-C10 alkenyl or alkynyl, and 1- (4-aminocyclohexyl) methylene.

**15.** The adduct according to claim 14, wherein said compound of formula (I) is represented by formula (II) below

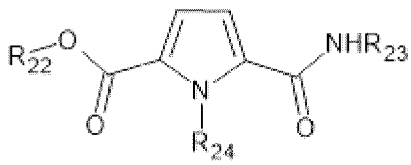
(II)

wherein said $R_{22}$ is selected from the group consisting of hydrogen and C1-C10 linear or branched alkyl group optionally substituted with one or more -OH groups; and said $R_{23}$-$R_{24}$ are the same and are selected from the group consisting of hydrogen, linear or branched C1-C10 alkyl, linear or branched C2-C10 alkenyl or alkynyl, aryl, linear or branched C1-C10 alkyl-aryl, linear or branched C2-C10 alkenyl-aryl, linear or branched C2-C10 alkynyl-aryl, heteroaryl, linear or branched C1-C10 alkyl-$NH_2$, linear or branched C1-C10 alkyl-SH, linear or branched C1-C10 alkyl-COOH, and linear or branched C1-C10 alkyl-$Si(OCH_2CH_3)_3$, wherein said alkyl groups are optionally substituted with one or more -OH groups.

**16.** The adduct according to any one of claims 14-15, wherein said $sp^2$ hybridized carbon allotrope is selected from the group consisting of: carbon black, graphene, 2 layer graphene, from 3 to 10 layer graphene, graphite, high surface area graphite, single wall or multiwall carbon nanotubes, carbon nanotubes of longitudinal or helicoid extension, nanocones, nanohorns, nanotoroids, fullerene and mixtures thereof.

**17.** Process for the preparation of an adduct according to any one of claims 14-16, comprising the steps of:

i) providing a mixture of a compound of formula (I) as defined in any one of claims 14-15 and a $sp^2$ hybridized carbon allotrope; and
ii) supplying energy to the mixture obtained in step i) to obtain an adduct; and
iii) optionally separating the adduct obtained.

**Patentansprüche**

**1.** Elastomerzusammensetzung, umfassend mindestens ein $sp^2$-hybridisiertes Kohlenstoffallotrop und eine Verbindung von Formel (I)

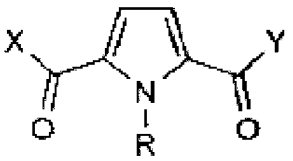

(I)

wobei X und Y unabhängig voneinander aus der Gruppe ausgewählt sind, bestehend aus:
-NHR' und OR", und
wobei R" aus der Gruppe ausgewählt ist, bestehend aus Wasserstoff, linearem oder verzweigtem C1-C10-Alkyl, linearem oder verzweigtem C2-C10-Alkenyl oder -Alkinyl, Aryl, linearem oder verzweigtem C1-C10-Alkyl-aryl, linearem oder verzweigtem C2-C10-Alkenyl-aryl, linearem oder verzweigtem C2-C10-Alkinyl-aryl und Heteroaryl; und
wobei R und R' unabhängig voneinander aus der Gruppe ausgewählt sind, bestehend aus: Wasserstoff, linearem oder verzweigtem C1-C10-Alkyl, linearem oder verzweigtem C2-C10-Alkenyl oder -Alkinyl, Aryl, linearem oder verzweigtem C1-C10-Alkyl-aryl, linearem oder verzweigtem C2-C10-Alkenyl-aryl, linearem oder verzweigtem C2-C10-Alkinyl-aryl, Heteroaryl und Carboxyl;
oder R und/oder R'

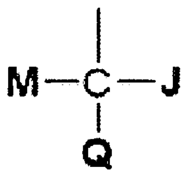

ist,
wobei M, Q und J unabhängig voneinander in einer Gruppe ausgewählt sind, bestehend aus Amin, Hydroxyl, Wasserstoff, linearem oder verzweigtem C1-C10-Alkyl und linearem oder verzweigtem C2-C10-Alkenyl oder -Alkinyl, oder in einer Gruppe, bestehend aus:

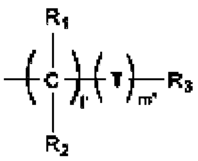 , 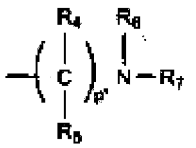 , 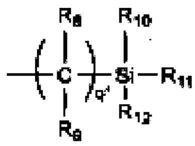 , 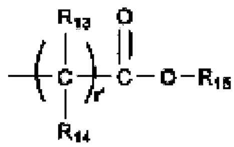

wobei $R_1$-$R_{15}$ unabhängig voneinander aus der Gruppe ausgewählt sind, bestehend aus: Wasserstoff, linearem oder verzweigtem C1-C10-Alkyl, linearem oder verzweigtem C2-C10-Alkenyl oder -Alkinyl, Aryl, linearem oder verzweigtem C1-C10-Alkyl-aryl, linearem oder verzweigtem C2-C10-Alkenyl-aryl, linearem oder verzweigtem C2-C10-Alkinyl-aryl, Heteroaryl und Carboxyl;
und wobei $R_{10}$-$R_{12}$ $-OCH_2-CH_3$ oder $-OCH_3$ sein können;
und wobei $R_{14}$ $-CH_2-SH$ oder $-CH_2-CH_2-S-CH_3$ sein kann;
und wobei T -O- oder -S- ist, und wenn T -S- ist, dann p' eine ganze Zahl von 1 bis 4 ist, wenn T -O- ist, dann p' eine ganze Zahl von 1 bis 2 ist;
und wobei l', p', q', r' unabhängig voneinander ganze Zahlen von 0 bis 12 sind;
oder R und/oder R'

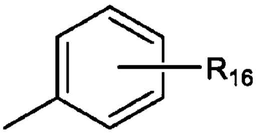

oder

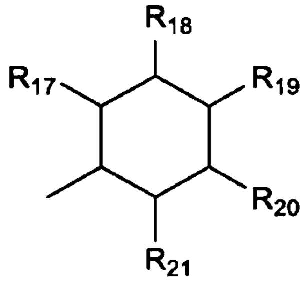

,

ist,
wobei $R_{16}$ aus der Gruppe ausgewählt ist, bestehend aus Wasserstoff, Alkyl, Aryl, Benzyl, Amin, Alkylamin, Arylamin, Benzylamin und Aminoaryl; und
$R_{17}$-$R_{21}$ unabhängig voneinander aus der Gruppe ausgewählt sind, bestehend aus: Wasserstoff, linearem oder verzweigtem C1-C10-Alkyl, linearem oder verzweigtem C2-C10-Alkenyl oder -Alkinyl, und 1-(4-Aminocyclohexyl)methylen.

**2.** Elastomerzusammensetzung nach Anspruch 1, wobei R und R' gleich sind.

**3.** Elastomerzusammensetzung nach Anspruch 1, wobei die Verbindung der Formel (I) durch die nachstehende Formel (II) dargestellt wird

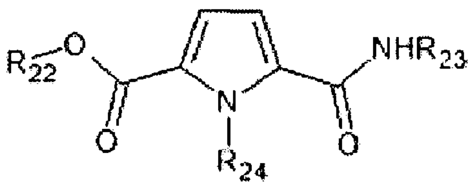

(II)

wobei das $R_{22}$ aus der Gruppe ausgewählt ist, bestehend aus Wasserstoff und einer linearen oder verzweigten C1-C10-Alkylgruppe, die gegebenenfalls mit einer oder mehreren -OH-Gruppen substituiert ist; und das $R_{23}$-$R_{24}$ gleich sind und aus der Gruppe ausgewählt sind, bestehend aus Wasserstoff, linearem oder verzweigtem C1-C10-Alkyl, linearem oder verzweigtem C2-C10-Alkenyl oder -Alkinyl, Aryl, linearem oder verzweigtem C1-C10-Alkyl-aryl, linearem oder verzweigtem C2-C10-Alkenyl-aryl, linearem oder verzweigtem C2-C10-Alkinyl-aryl, Heteroaryl, linearem oder verzweigtem C1-C10-Alkyl-$NH_2$, linearem oder verzweigtem C1-C10-Alkyl-SH, linearem oder verzweigtem C1-C10-Alkyl-COOH und linearem oder verzweigtem C1-C10-Alkyl-$Si(OCH_2CH_3)_3$, wobei die Alkylgruppen gegebenenfalls mit einer oder mehreren -OH-Gruppen substituiert sind.

**4.** Elastomerzusammensetzung nach einem der Ansprüche 1-3, wobei das $sp^2$-hybridisierte Kohlenstoffallotrop aus der Gruppe ausgewählt ist, bestehend aus: Ruß, Graphen, 2-Schicht-Graphen, 3 bis 10-Schicht-Graphen, Graphit, Graphit mit hoher Oberfläche, einwandigen oder mehrwandigen Kohlenstoffnanoröhrchen, Kohlenstoffnanoröhrchen mit longitudinaler oder helicoider Ausdehnung, Nanokegeln, Nanohörnern, Nanotoroiden, Fulleren und Mischungen davon.

**5.** Elastomerzusammensetzung nach einem der Ansprüche 1 bis 4, umfassend mindestens ein ungesättigtes Elastomer, das in der Gruppe ausgewählt ist, bestehend aus: Poly(1,4-cis-isopren), Naturkautschuk oder synthetischem Polymer, Poly(3,4-isopren), Poly(butadien), Poly(butadien) mit einem hohen Gehalt an 1,4-cis-Einheiten, Isopren/Isobuten-Copolymeren, halogenierten Isopren/Isobuten-Copolymeren, halogeniertem Butylkautschuk, Chlorbutylkautschuk, Brombutylkautschuk, 1,3-Butadien/Acrylnitril-Copolymeren, Styrol/1,3-Butadien-Copolymeren, Styrol/Isopren/1,3-Butadien-Copolymeren und Styrol/1,3-Butadien/Acrylnitril-Copolymeren und Mischungen davon.

**6.** Elastomerzusammensetzung nach Anspruch 5, ferner umfassend mindestens ein Elastomer aus einem oder mehreren Monoolefinen, gegebenenfalls enthaltend ein Dien.

**7.** Elastomerzusammensetzung nach einem der Ansprüche 1 bis 6, ferner umfassend zusätzliche verstärkende Füllstoffe, die aus der Gruppe ausgewählt sind, umfassend: Ruß, Siliciumdioxid, Schichtsilikate, Mischoxide von Aluminium und Magnesium mit lamellarer Struktur, Tonerde und Silicoaluminate und Mischungen davon.

8. Elastomerzusammensetzung nach Anspruch 7, wobei die zusätzlichen verstärkenden Füllstoffe aus der Gruppe ausgewählt sind, bestehend aus: Siliciumdioxid, Ruß und Mischungen davon.

9. Elastomerzusammensetzung nach einem der Ansprüche 1 bis 8, ferner umfassend ein oder mehrere Vulkanisierungsmittel, gegebenenfalls umfassend einen Aktivator und/oder einen Beschleuniger, und ein oder mehrere Additive, die aus Antioxidantien, Alterungsschutzmitteln, Weichmachern, Klebstoffen, Anti-Ozon-Mitteln, modifizierenden Harzen und Mischungen davon ausgewählt sind.

10. Verfahren für die Herstellung einer Elastomerzusammensetzung nach einem der Ansprüche 1 bis 9, umfassend die Schritte:

a) Bereitstellen eines oder mehrerer Elastomere, mindestens eines $sp^2$-hybridisierten Kohlenstoffallotrops, einer Verbindung der Formel (I) wie in einem der Ansprüche 1 bis 3 definiert, gegebenenfalls eines oder mehrerer zusätzlicher verstärkender Füllstoffe, gegebenenfalls eines oder mehrerer Additive und gegebenenfalls eines oder mehrerer Vulkanisierungsmittel; und
b) Durchführen mindestens eines Mischschritts.

11. Verfahren nach Anspruch 10, wobei die Verbindung der Formel (I) und das mindestens eine $sp^2$-hybridisierte Kohlenstoffallotrop als ein vorgeformtes Addukt bereitgestellt werden.

12. Reifen für Fahrzeugräder, umfassend mindestens ein Strukturelement, umfassend ein vernetztes Elastomermaterial, das durch Vernetzen einer Elastomerzusammensetzung nach einem der Ansprüche 1 bis 9 erhalten wird.

13. Reifen nach Anspruch 12, wobei das Strukturelement in der Gruppe ausgewählt ist, bestehend aus: Laufstreifen, Seitenwand, Seitenwandeinsatz, Schichten aus Elastomermaterial radial innen bezüglich des Laufstreifens, wobei die radial inneren Schichten vorzugsweise ausgewählt sind aus: Felgenband und Mini-Seitenwand, Wulststrukturen und Gummibeschichtung der Textilien und der Metalle.

14. Addukt zwischen einem $sp^2$-hybridisierten Kohlenstoffallotrop und einer Verbindung der Formel (I)

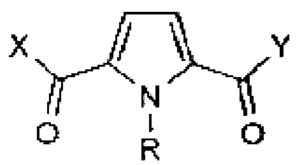

(I)

wobei X und Y unabhängig voneinander aus der Gruppe ausgewählt sind, bestehend aus:
-NHR' und OR", und
wobei R" aus der Gruppe ausgewählt ist, bestehend aus Wasserstoff, linearem oder verzweigtem C1-C10-Alkyl, linearem oder verzweigtem C2-C10-Alkenyl oder -Alkinyl, Aryl, linearem oder verzweigtem C1-C10-Alkyl-aryl, linearem oder verzweigtem C2-C10-Alkenyl-aryl, linearem oder verzweigtem C2-C10-Alkinyl-aryl und Heteroaryl; und
wobei R und R' unabhängig voneinander aus der Gruppe ausgewählt sind, bestehend aus: Wasserstoff, linearem oder verzweigtem C1-C10-Alkyl, linearem oder verzweigtem C2-C10-Alkenyl oder -Alkinyl, Aryl, linearem oder verzweigtem C1-C10-Alkyl-aryl, linearem oder verzweigtem C2-C10-Alkenyl-aryl, linearem oder verzweigtem C2-C10-Alkinyl-aryl, Heteroaryl und Carboxyl;
oder R und/oder R'

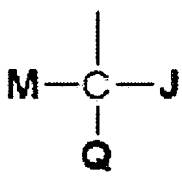

ist,

wobei M, Q und J unabhängig voneinander in einer Gruppe ausgewählt sind, bestehend aus Amin, Hydroxyl, Wasserstoff, linearem oder verzweigtem C1-C10-Alkyl und linearem oder verzweigtem C2-C10-Alkenyl oder -Alkinyl, oder in einer Gruppe, bestehend aus:

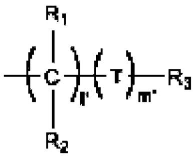,
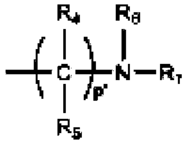,
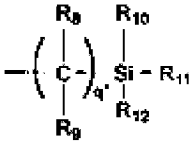,
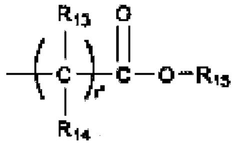

wobei $R_1$-$R_{15}$ unabhängig voneinander aus der Gruppe ausgewählt sind, bestehend aus: Wasserstoff, linearem oder verzweigtem C1-C10-Alkyl, linearem oder verzweigtem C2- C10-Alkenyl oder -Alkinyl, Aryl, linearem oder verzweigtem C1-C10-Alkyl-aryl, linearem oder verzweigtem C2-C10-Alkenyl-aryl, linearem oder verzweigtem C2-C10-Alkinyl-aryl, Heteroaryl und Carboxyl;
und wobei $R_{10}$-$R_{12}$ $-OCH_2-CH_3$ oder $-OCH_3$ sein können;
und wobei $R_{14}$ $-CH_2-SH$ oder $-CH_2-CH_2-S-CH_3$ sein kann;
und wobei T -O- oder -S- ist, und wenn T -S- ist, dann p' eine ganze Zahl von 1 bis 4 ist, wenn T -O- ist, dann p' eine ganze Zahl von 1 bis 2 ist;
und wobei l', p', q', r' unabhängig voneinander ganze Zahlen von 0 bis 12 sind;
oder R und/oder R'

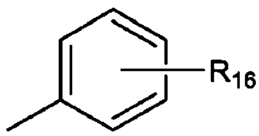

oder

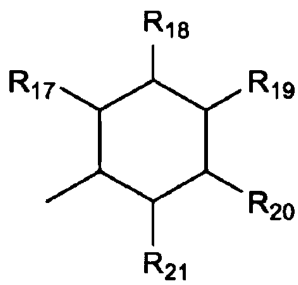,

ist,
wobei $R_{16}$ aus der Gruppe ausgewählt ist, bestehend aus Wasserstoff, Alkyl, Aryl, Benzyl, Amin, Alkylamin, Arylamin, Benzylamin und Aminoaryl; und
$R_{17}$-$R_{21}$ unabhängig voneinander aus der Gruppe ausgewählt sind, bestehend aus: Wasserstoff, linearem oder verzweigtem C1-C10-Alkyl, linearem oder verzweigtem C2-C10-Alkenyl oder -Alkinyl, und 1-(4-Aminocyclohexyl)methylen.

**15.** Addukt nach Anspruch 14, wobei die Verbindung der Formel (I) durch die nachstehende Formel (II) dargestellt wird

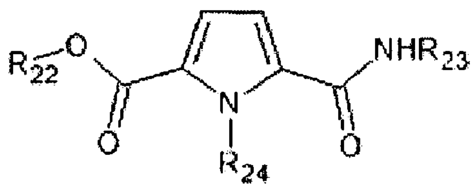
(II)

wobei das $R_{22}$ aus der Gruppe ausgewählt ist, bestehend aus Wasserstoff und einer linearen oder verzweigten C1-C10-Alkylgruppe, die gegebenenfalls mit einer oder mehreren -OH-Gruppen substituiert ist; und das $R_{23}$-$R_{24}$ gleich

sind und aus der Gruppe ausgewählt sind, bestehend aus Wasserstoff, linearem oder verzweigtem C1-C10-Alkyl, linearem oder verzweigtem C2-C10-Alkenyl oder -Alkinyl, Aryl, linearem oder verzweigtem C1-C10-Alkyl-aryl, linearem oder verzweigtem C2-C10-Alkenyl-aryl, linearem oder verzweigtem C2-C10-Alkinyl-aryl, Heteroaryl, linearem oder verzweigtem C1-C10-Alkyl-$NH_2$, linearem oder verzweigtem C1-C10-Alkyl-SH, linearem oder verzweigtem C1-C10-Alkyl-COOH und linearem oder verzweigtem C1-C10-Alkyl-$Si(OCH_2CH_3)_3$, wobei die Alkylgruppen gegebenenfalls mit einer oder mehreren -OH-Gruppen substituiert sind.

**16.** Addukt nach einem der Ansprüche 14 bis 15, wobei das $sp^2$-hybridisierte Kohlenstoffallotrop aus der Gruppe ausgewählt ist, bestehend aus: Ruß, Graphen, 2-Schicht-Graphen, 3 bis 10-Schicht-Graphen, Graphit, Graphit mit hoher Oberfläche, einwandigen oder mehrwandigen Kohlenstoffnanoröhrchen, Kohlenstoffnanoröhrchen mit longitudinaler oder helicoider Ausdehnung, Nanokegeln, Nanohörnern, Nanotoroiden, Fulleren und Mischungen davon.

**17.** Verfahren für die Herstellung eines Addukts nach einem der Ansprüche 14 bis 16, umfassend die Schritte:

i) Bereitstellen einer Mischung einer Verbindung der Formel (I) wie in einem der Ansprüche 14 bis 15 definiert und eines $sp^2$-hybridisierten Kohlenstoffallotrops; und
ii) Zuführen von Energie zu der in Schritt i) erhaltenen Mischung, um ein Addukt zu erhalten; und
iii) gegebenenfalls Abtrennen des erhaltenen Addukts.

**Revendications**

**1.** Composition élastomère comprenant au moins un allotrope de carbone à hybridation $sp^2$ et un composé de formule (I)

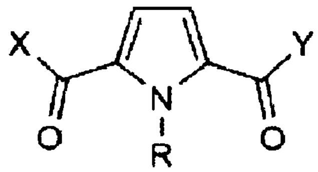

(I)

dans laquelle X et Y sont indépendamment choisis dans le groupe constitué de :
-NHR' et OR", et
dans laquelle R" est choisi dans le groupe constitué d'hydrogène, alkyle en C1 à C10 linéaire ou ramifié, alcényle ou alcynyle en C2 à C10 linéaire ou ramifié, aryle, alkyl-aryle en C1 à C10 linéaire ou ramifié, alcényl-aryle en C2 à C10 linéaire ou ramifié, alcynyl-aryle en C2 à C10 linéaire ou ramifié et hétéroaryle ; et
dans laquelle R et R' sont indépendamment choisis dans le groupe constitué de : hydrogène, alkyle en C1 à C10 linéaire ou ramifié, alcényle ou alcynyle en C2 à C10 linéaire ou ramifié, aryle, alkyl-aryle en C1 à C10 linéaire ou ramifié, alcényl-aryle en C2 à C10 linéaire ou ramifié, alcynyl-aryle en C2 à C10 linéaire ou ramifié, hétéroaryle et carboxyle ;
ou R et/ou R' sont

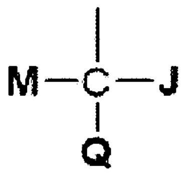

dans laquelle M, Q et J sont indépendamment choisis dans un groupe constitué d'amine, hydroxyle, hydrogène, alkyle en C1 à C10 linéaire ou ramifié et alcényle ou alcynyle en C2 à C10 linéaire ou ramifié, ou dans un groupe constitué de :

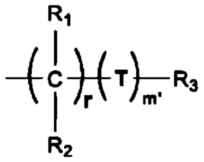 , 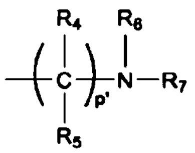 , 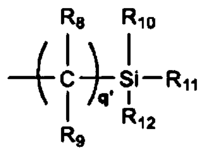 , 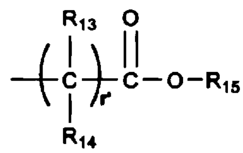 ,

dans laquelle $R_1$ à $R_{15}$ sont indépendamment choisis dans le groupe constitué de : hydrogène, alkyle en C1 à C10 linéaire ou ramifié, alcényle ou alcynyle en C2 à C10 linéaire ou ramifié, aryle, alkyl-aryle en C1 à C10 linéaire ou ramifié, alcényl-aryle en C2 à C10 linéaire ou ramifié, alcynyl-aryle en C2 à C10 linéaire ou ramifié, hétéroaryle et carboxyle ;
et dans laquelle $R_{10}$ à $R_{12}$ peuvent être $-OCH_2-CH_3$ ou $-OCH_3$ ;
et dans laquelle $R_{14}$ peut être $-CH_2-SH$ ou $-CH_2-CH_2-S-CH_3$ ;
et dans laquelle T est -O- ou -S-, et lorsque T est -S-, p' est alors un nombre entier allant de 1 à 4, lorsque T est -O-, p' est alors un nombre entier allant de 1 à 2 ;
et dans laquelle l', p', q', r' sont indépendamment des nombres entiers allant de 0 à 12 ;
ou R et/ou R' sont

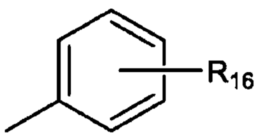 ou 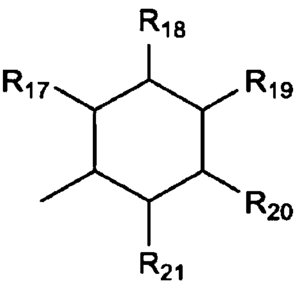 ,

dans laquelle $R_{16}$ est choisi dans le groupe constitué d'hydrogène, alkyle, aryle, benzyle, amine, alkylamine, arylamine, benzylamine et aminoaryle ; et
$R_{17}$ à $R_{21}$ sont indépendamment choisis dans le groupe constitué de : hydrogène, alkyle en C1 à C10 linéaire ou ramifié, alcényle ou alcynyle en C2 à C10 linéaire ou ramifié, et 1-(4-aminocyclohexyl)méthylène.

2. Composition élastomère selon la revendication 1, dans laquelle R et R' sont identiques.

3. Composition élastomère selon la revendication 1, dans laquelle ledit composé de formule (I) est représenté par la formule (II) ci-dessous

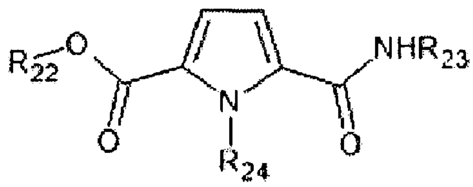

(II)

dans laquelle ledit $R_{22}$ est choisi dans le groupe constitué d'hydrogène et d'un groupe alkyle en C1 à C10 linéaire ou ramifié facultativement substitué par un ou plusieurs groupes -OH ; et lesdits $R_{23}$ à $R_{24}$ sont identiques et sont choisis dans le groupe constitué d'hydrogène, alkyle en C1 à C10 linéaire ou ramifié, alcényle ou alcynyle en C2 à C10 linéaire ou ramifié, aryle, alkyl-aryle en C1 à C10 linéaire ou ramifié, alcényl-aryle en C2 à C10 linéaire ou ramifié, alcynyl-aryle en C2 à C10 linéaire ou ramifié, hétéroaryle, alkyl-$NH_2$ en C1 à C10 linéaire ou ramifié, alkyl-SH en C1 à C10 linéaire ou ramifié, alkyl-COOH en C1 à C10 linéaire ou ramifié, et alkyl-$Si(OCH_2CH_3)_3$ en C1 à C10 linéaire ou ramifié, dans laquelle lesdits groupes alkyle sont facultativement substitués par un ou plusieurs groupes -OH.

4. Composition élastomère selon l'une quelconque des revendications 1 à 3, dans laquelle ledit allotrope de carbone à hybridation $sp^2$ est choisi dans le groupe constitué de : noir de carbone, graphène, graphène à 2 couches, graphène de 3 à 10 couches, graphite, graphite à haute surface spécifique, nanotubes de carbone à paroi unique ou à parois multiples, nanotubes de carbone d'extension longitudinale ou hélicoïdale, nanocônes, nanocornes, nanotoroïdes,

fullerènes et mélanges de ceux-ci.

5. Composition élastomère selon l'une quelconque des revendications 1 à 4, comprenant au moins un élastomère insaturé choisi dans le groupe constitué de : poly(1,4-cis-isoprène), caoutchouc naturel ou polymère synthétique, poly(3,4-isoprène), poly(butadiène), poly(butadiène) avec une teneur élevée en motifs 1,4-cis, copolymères d'isoprène/isobutène, copolymères d'isoprène halogéné/isobutène, caoutchouc butyle halogéné, caoutchouc chlorobutyle, caoutchouc bromobutyle, copolymères de 1,3-butadiène/acrylonitrile, copolymères de styrène/1,3-butadiène, copolymères de styrène/isoprène/1,3-butadiène, et copolymères de styrène/1,3-butadiène/acrylonitrile, et mélanges de ceux-ci.

6. Composition élastomère selon la revendication 5, comprenant en outre au moins un élastomère d'une ou plusieurs mono-oléfines, contenant facultativement un diène.

7. Composition élastomère selon l'une quelconque des revendications 1 à 6 comprenant en outre des charges renforçantes supplémentaires choisies dans le groupe comprenant : noir de carbone, silice, silicate en couches, oxydes mixtes d'aluminium et de magnésium avec une structure lamellaire, alumine et silico-aluminates et mélanges de ceux-ci.

8. Composition élastomère selon la revendication 7, dans laquelle lesdites charges renforçantes supplémentaires sont choisies dans le groupe constitué de : silice, noir de carbone et mélanges de ceux-ci.

9. Composition élastomère selon l'une quelconque des revendications 1 à 8, comprenant en outre un ou plusieurs agents de vulcanisation, comprenant facultativement un activateur et/ou un accélérateur, et un ou plusieurs additifs choisis parmi antioxydants, agents anti-vieillissement, plastifiants, adhésifs, agents anti-ozone, résines de modification, et mélanges de ceux-ci.

10. Procédé destiné à la préparation d'une composition élastomère selon l'une quelconque des revendications 1 à 9, comprenant les étapes consistant à :

a) fournir un ou plusieurs élastomères, au moins un allotrope de carbone à hybridation $sp^2$, un composé de formule (I) tel que défini dans l'une quelconque des revendications 1 à 3, facultativement une ou plusieurs charges renforçantes supplémentaires, facultativement un ou plusieurs additifs, et facultativement un ou plusieurs agents de vulcanisation ; et
b) mettre en œuvre au moins une étape de mélange.

11. Procédé selon la revendication 10, dans lequel ledit composé de formule (I) et ledit au moins un allotrope de carbone à hybridation $sp^2$ sont fournis en tant qu'adduit préformé.

12. Pneu destiné à des roues de véhicule comprenant au moins un élément structural comprenant un matériau élastomère réticulé obtenu par réticulation d'une composition élastomère selon l'une quelconque des revendications 1 à 9.

13. Pneu selon la revendication 12, dans lequel ledit élément structural est choisi dans le groupe constitué de : bande de roulement, flanc, insert de flanc, couches de matériau élastomère radialement internes par rapport à ladite bande de roulement, dans lequel lesdites couches radialement internes sont choisies de préférence parmi : coussinet et mini-flanc, structures de talon, ainsi que le revêtement en caoutchouc des textiles et des métaux.

14. Adduit entre un allotrope de carbone à hybridation $sp^2$ et un composé de formule (I)

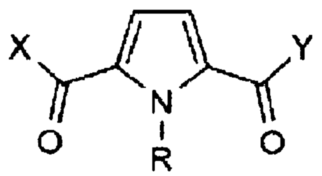

(I)

dans lequel X et Y sont indépendamment choisis dans le groupe constitué de :

-NHR' et OR", et

dans lequel R" est choisi dans le groupe constitué d'hydrogène, alkyle en C1 à C10 linéaire ou ramifié, alcényle ou alcynyle en C2 à C10 linéaire ou ramifié, aryle, alkyl-aryle en C1 à C10 linéaire ou ramifié, alcényl-aryle en C2 à C10 linéaire ou ramifié, alcynyl-aryle en C2 à C10 linéaire ou ramifié, et hétéroaryle ; et

dans lequel R et R' sont indépendamment choisis dans le groupe constitué de : hydrogène, alkyle en C1 à C10 linéaire ou ramifié, alcényle ou alcynyle en C2 à C10 linéaire ou ramifié, aryle, alkyl-aryle en C1 à C10 linéaire ou ramifié, alcényl-aryle en C2 à C10 linéaire ou ramifié, alcynyl-aryle en C2 à C10 linéaire ou ramifié, hétéroaryle et carboxyle ;

ou R et/ou R' sont

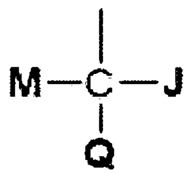

dans lequel M, Q et J sont indépendamment choisis dans un groupe constitué d'amine, hydroxyle, hydrogène, alkyle en C1 à C10 linéaire ou ramifié et alcényle ou alcynyle en C2 à C10 linéaire ou ramifié, ou dans un groupe constitué de

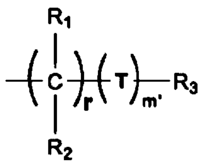 , 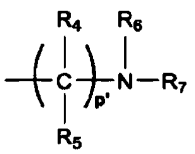 , 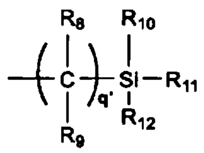 , 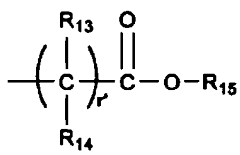 ,

dans lequel $R_1$ à $R_{15}$ sont indépendamment choisis dans le groupe constitué de : hydrogène, alkyle en C1 à C10 linéaire ou ramifié, alcényle ou alcynyle en C2 à C10 linéaire ou ramifié, aryle, alkyl-aryle en C1 à C10 linéaire ou ramifié, alcényl-aryle en C2 à C10 linéaire ou ramifié, alcynyl-aryle en C2 à C10 linéaire ou ramifié, hétéroaryle et carboxyle ;

et dans lequel $R_{10}$ à $R_{12}$ peuvent être $-OCH_2-CH_3$ ou $-OCH_3$ ;

et dans lequel $R_{14}$ peut être $-CH_2-SH$ ou $-CH_2-CH_2-S-CH_3$ ;

et dans lequel T est -O- ou -S-, et lorsque T est -S-, p' est alors un nombre entier allant de 1 à 4, lorsque T est -O-, p' est alors un nombre entier allant de 1 à 2 ;

et dans lequel l', p', q', r' sont indépendamment des nombres entiers allant de 0 à 12 ;

ou R et/ou R' sont

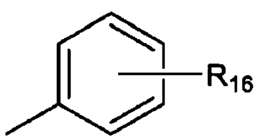 ou 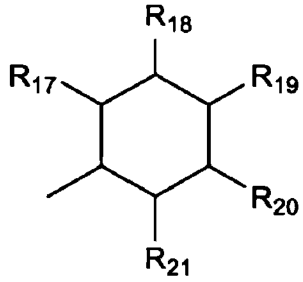 ,

dans lequel $R_{16}$ est choisi dans le groupe constitué d'hydrogène, alkyle, aryle, benzyle, amine, alkylamine, arylamine, benzylamine et aminoaryle ; et

$R_{17}$ à $R_{21}$ sont indépendamment choisis dans le groupe constitué de : hydrogène, alkyle en C1 à C10 linéaire ou ramifié, alcényle ou alcynyle en C2 à C10 linéaire ou ramifié, et 1-(4-aminocyclohexyl)méthylène.

**15.** Adduit selon la revendication 14, dans lequel ledit composé de formule (I) est représenté par la formule (II) ci-dessous

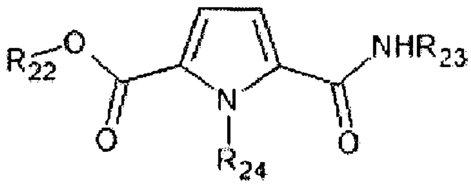

(II)

dans lequel ledit $R_{22}$ est choisi dans le groupe constitué d'hydrogène et d'un groupe alkyle en C1 à C10 linéaire ou ramifié facultativement substitué par un ou plusieurs groupes -OH ; et lesdits $R_{23}$ à $R_{24}$ sont identiques et sont choisis dans le groupe constitué d'hydrogène, alkyle en C1 à C10 linéaire ou ramifié, alcényle ou alcynyle en C2 à C10 linéaire ou ramifié, aryle, alkyl-aryle en C1 à C10 linéaire ou ramifié, alcényl-aryle en C2 à C10 linéaire ou ramifié, alcynyl-aryle en C2 à C10 linéaire ou ramifié, hétéroaryle, alkyl-$NH_2$ en C1 à C10 linéaire ou ramifié, alkyl-SH en C1 à C10 linéaire ou ramifié, alkyl-COOH en C1 à C10 linéaire ou ramifié, et alkyl-$Si(OCH_2CH_3)_3$ en C1 à C10 linéaire ou ramifié, dans lequel lesdits groupes alkyle sont facultativement substitués par un ou plusieurs groupes -OH.

**16.** Adduit selon l'une quelconque des revendications 14 à 15, dans lequel ledit allotrope de carbone à hybridation $sp^2$ est choisi dans le groupe constitué de : noir de carbone, graphène, graphène à 2 couches, graphène de 3 à 10 couches, graphite, graphite à haute surface spécifique, nanotubes de carbone à paroi unique ou à parois multiples, nanotubes de carbone d'extension longitudinale ou hélicoïdale, nanocônes, nanocornes, nanotoroïdes, fullerènes et mélanges de ceux-ci.

**17.** Procédé destiné à la préparation d'un adduit selon l'une quelconque des revendications 14 à 16, comprenant les étapes consistant à :

i) fournir un mélange d'un composé de formule (I) tel que défini dans l'une quelconque des revendications 14 à 15 et d'un allotrope de carbone à hybridation $sp^2$ ; et
ii) alimenter en énergie le mélange obtenu à l'étape i) pour obtenir un adduit ; et
iii) facultativement séparer l'adduit obtenu.

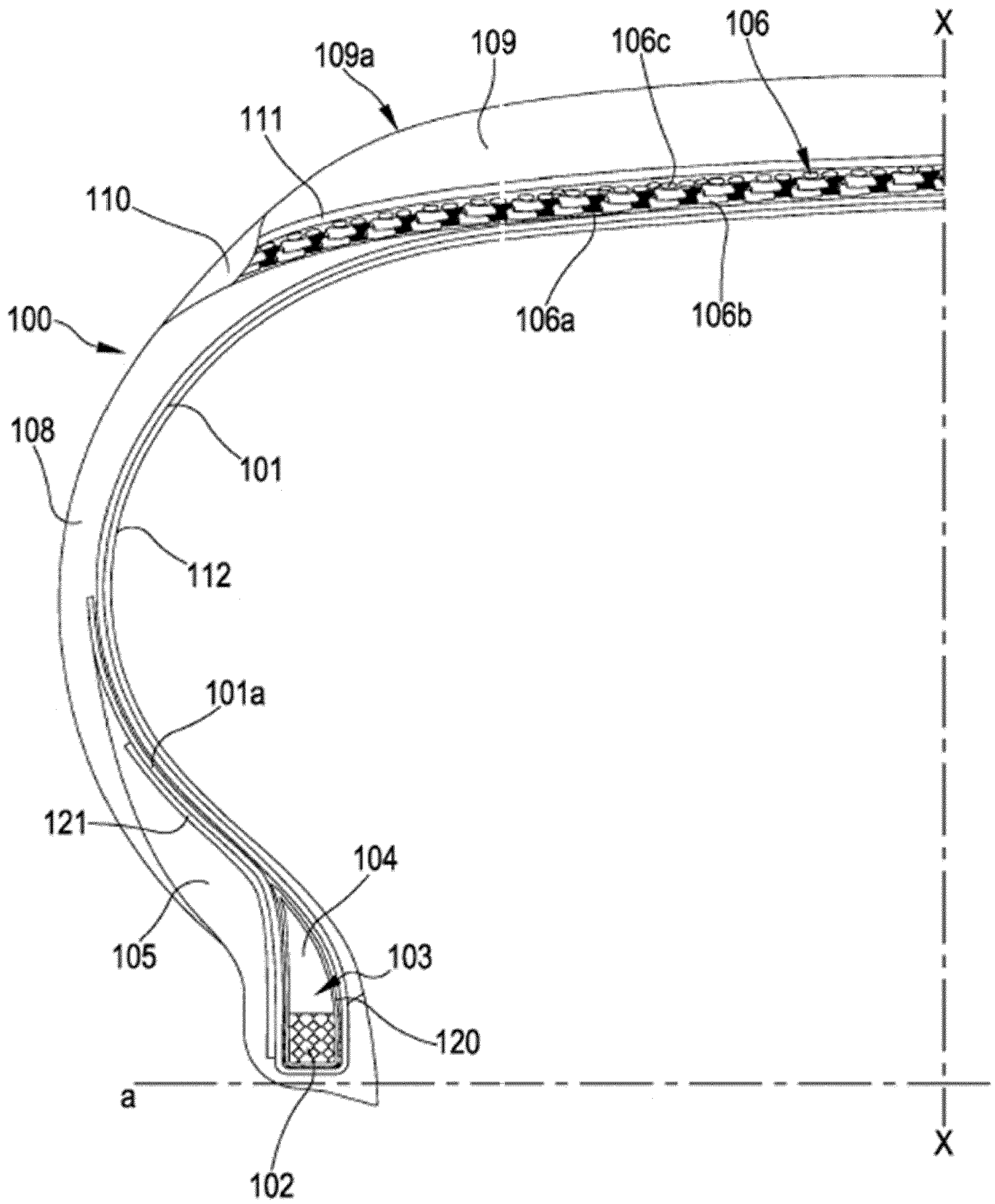
106
X
106c
109
109a
111
110
100
106a
106b
108
101
112
101a
121
104
105
103
120
a
X
102

Fig. 1

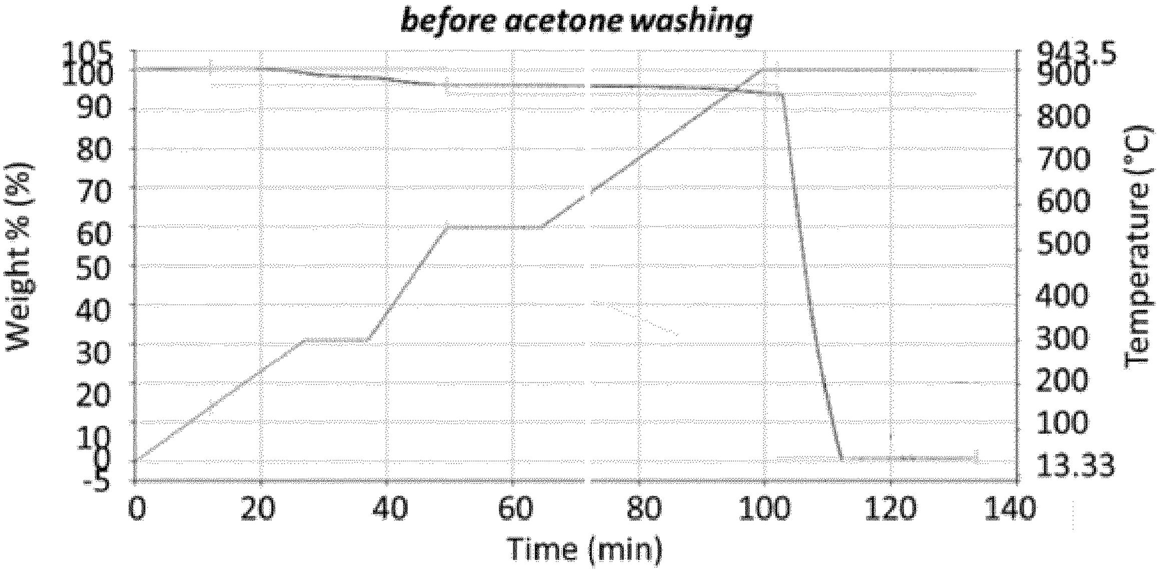
before acetone washing
Weight % (%)
105
100
90
80
70
60
50
40
30
20
10
0
-5
Temperature (°C)
943.5
900
800
700
600
500
400
300
200
100
13.33
0
20
40
60
80
100
120
140
Time (min)

Fig. 2a

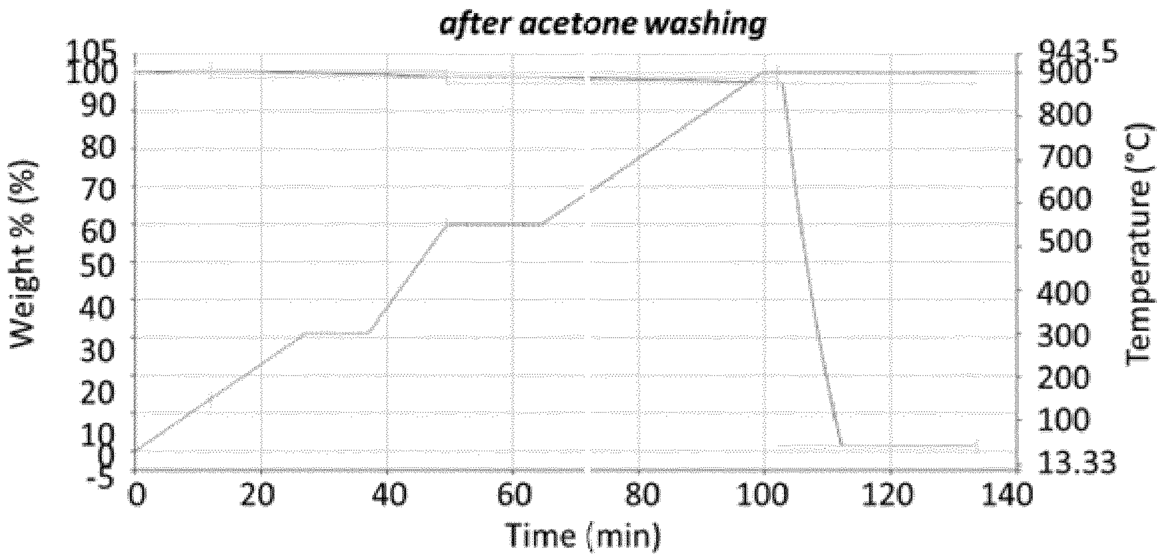
after acetone washing
Weight % (%)
105
100
90
80
70
60
50
40
30
20
10
0
-5
Temperature (°C)
943.5
900
800
700
600
500
400
300
200
100
13.33
0
20
40
60
80
100
120
140
Time (min)

Fig. 2b

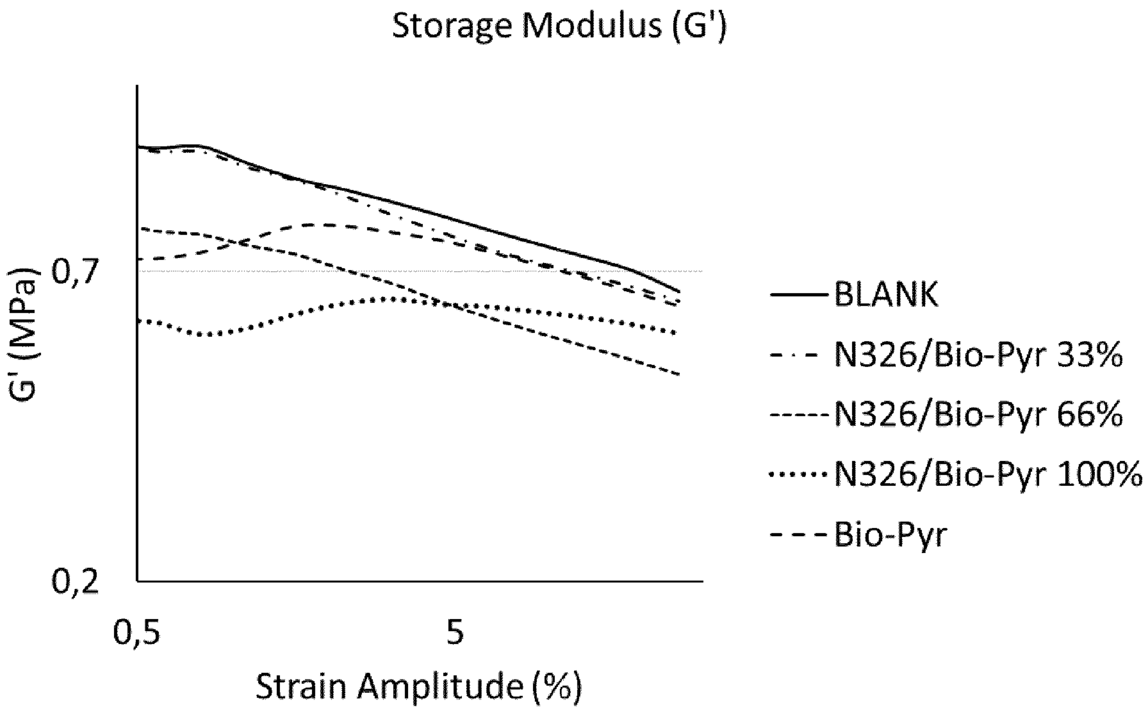
Storage Modulus (G')
G' (MPa)
0,7
0,2
0,5
5
Strain Amplitude (%)
BLANK
N326/Bio-Pyr 33%
N326/Bio-Pyr 66%
N326/Bio-Pyr 100%
Bio-Pyr

Fig. 3

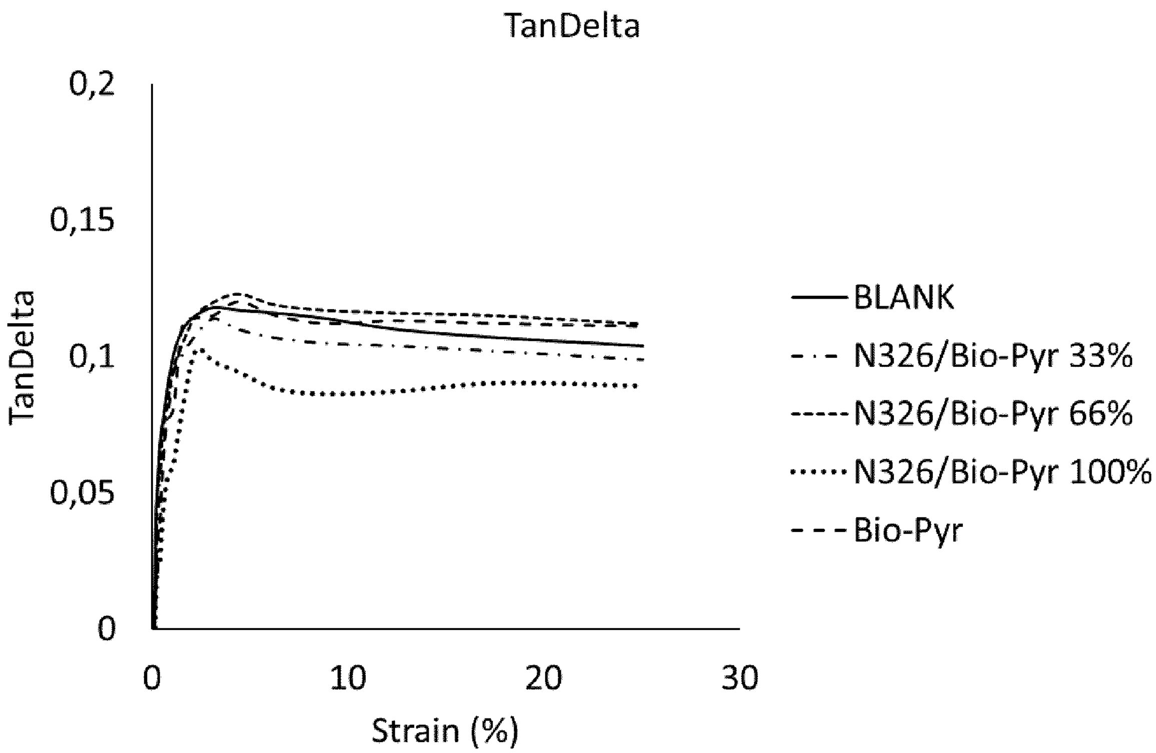
TanDelta
0,2
0,15
0,1
0,05
0
TanDelta
0
10
20
30
Strain (%)
BLANK
N326/Bio-Pyr 33%
N326/Bio-Pyr 66%
N326/Bio-Pyr 100%
Bio-Pyr

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016050887 A
- WO 2020225595 A1
- WO 2020222103 A